Europäisches Patentamt

European Patent Office

Office européen des brevets

(19)

(11)  EP 0 790 821 B1

(12)  **EUROPÄISCHE PATENTSCHRIFT**

(45) Veröffentlichungstag und Bekanntmachung des Hinweises auf die Patenterteilung:
**06.10.2004  Patentblatt 2004/41**

(51) Int Cl.[7]: **A61K 9/10**, A61K 9/14

(21) Anmeldenummer: **95938439.7**

(22) Anmeldetag: **09.11.1995**

(86) Internationale Anmeldenummer:
**PCT/EP1995/004401**

(87) Internationale Veröffentlichungsnummer:
**WO 1996/014830 (23.05.1996 Gazette 1996/23)**

(54) **VERFAHREN ZUR HERSTELLUNG VON PHARMAZEUTISCHEN NANOSUSPENSIONEN**

PROCESS FOR THE PREPARATION OF PHARMACEUTICAL NANOSUSPENSIONS

PROCEDE POUR LA PREPARATION DE NANOSUSPENSIONS PHARMACEUTIQUES

(84) Benannte Vertragsstaaten:
**AT BE CH DE DK ES FR GB GR IE IT LI LU MC NL PT SE**
Benannte Erstreckungsstaaten:
**LT LV SI**

(30) Priorität: **11.11.1994  DE 4440337**

(43) Veröffentlichungstag der Anmeldung:
**27.08.1997  Patentblatt 1997/35**

(73) Patentinhaber: **JAGOTEC AG**
**4132 Muttenz (CH)**

(72) Erfinder:
  • **MÜLLER, Rainer, H.**
    **D-12161 Berlin (DE)**
  • **BECKER, Robert**
    **D-88400 Biberach (DE)**
  • **KRUSS, Bernd**
    **D-88454 Hochdorf (DE)**
  • **PETERS, Katrin**
    **D-10559 Berlin (DE)**

(74) Vertreter: **Zimmermann, Hans, Dr. et al**
**A. Braun Braun Héritier Eschmann AG**
**Patentanwälte VSP**
**Postfach 160**
**4003 Basel (CH)**

(56) Entgegenhaltungen:
**EP-A- 0 361 928          EP-A- 0 600 528**
**WO-A-90/06746          WO-A-93/18752**
**WO-A-94/14426          WO-A-94/20072**
**DE-A- 4 217 842          US-A- 4 879 308**

  • **PROC. INT. SYMP. CONTROLLED RELEASE BIOACT. MATER., Bd. 22, 30.Juli 1995 - 2.August 1995 Seiten 574-575, XP 000566632 MUELLER R.H. ET AL 'nanosuspension for the iv administration of poorly soluble drugs stability during sterilization and long term storage'**
  • **EUROPEAN J. OF PHARM. AND BIOPHARM., Bd. 40, Nr. 3, Juni 1994 STUTTGART, Seiten 157-160, XP 000450221 BOCK T. ET AL 'high pressure homogenisation of parenteral fat emulsions - influence of process parametres on emulsion quality'**

EP 0 790 821 B1

**Beschreibung**

Beschreibung der Erfindung

1. Bereich der Erfindung

Die Erfindung betrifft ein Verfahren, das die Herstellung von Arneistoffträgern, umfassend Teilchen aus in Wasser unlöslichen oder nur wenig löslichen, reinen, bei Raumtemperatur festen Wirkstoffen, mit einem mittleren Durchmesser von 10 - 1000 nm bei gleichzeitig niedrigem Gehalt an Mikropartikeln in der Teilchenpopulation gestattet. Es ermöglicht die Herstellung von Arneistoffträgern aus reinem Wirkstoff mit hoher Sättigungslöslichkeit und hoher Lösungsgeschwindigkeit, deren physikalische Stabilisierung - insbesondere auch unter Verwendung sehr niedriger Tensidund Stabilisatorkonzentrationen - und neben anderen Applikationsformen auch deren intravenöse Injizierbarkeit.

2. Definition und Vorteile von Nanosuspensionen

Definition der Nanosuspension im Sinne der Erfindung:

Disperses System fest in flüssig oder fest in halbfest, wobei die dispergierte Phase aus reinem Wirkstoff oder einem Wirkstoffgemisch besteht. Der mittlere Durchmesser der dispergierten Phase liegt zwischen 10 nm und 1000 nm (bestimmt mit Photonenkorrelationsspektroskopie), wobei die Verteilung der Population recht eng ist, das heisst, der Anteil an Mikropartikeln in der Teilchenpopulation ist sehr gering. Die Nanosuspension kann tensidfrei sein, aber auch Tenside oder Stabilisatoren oder beide enthalten. Die Nanosuspension kann auch lyophilisiert oder sprühgetrocknet sein, auch können die Nanopartikel einer Nanosuspension in eine feste Trägermatrix eingearbeitet sein.

Vorteile von Nanosuspensionen

Die Herstellung von Arzneistoffpartikeln mit einer Größe im Nanometerbereich hat viele Vorteile aus pharmazeutische-technologischer, biopharmazeutischer, pharmakologischer und medizinischer Sicht. Einige davon sind:

1. Die Lösungsgeschwindigkeit steigt mit Vergrößerung der Partikeloberfläche entsprechend dem Gesetz von Noyes-Whitney. Dadurch erhöht sich die Anflutungsgeschwindigkeit von Wirkstoffen, der maximale Plasmaspiegel wird schneller erreicht (z.B. orale oder i.v. Applikation einer Nanosuspension). Herstellung von Nanosuspensionen ist somit für alle Substanzen interessant, bei denen die Auflösungsgeschwindigkeit der bestimmende Faktor für die Bioverfügbarkeit ist.

2. Die intravenöse Applikation schwerlöslicher Wirkstoffe kann durch Nanosuspensionen ermöglicht werden. Immer mehr neu entwickelte Arzneistoffe besitzen eine sehr niedrige Löslichkeit oder sind fast unlöslich, und zwar gleichzeitig in Wasser und organischen Lösungsmitteln. Eine pharmakologische Testung ist nach oraler oder i. m. Applikation aufgrund der durch die niedrige Löslichkeit bedingten geringen Bioverfügbarkeit nicht möglich. Intravenöse Injektion scheidet aufgrund Fehlens eines geeigneten Lösungsmittelgemiches aus. Als Nanosuspension kann der Wirkstoff ohne Blockade von Blutkapillaren injiziert werden. In dem im Vergleich zum Injektionsvolumen relativ großen Blutvolumen (z.B. 20 ml zu 6 l) kommt es dann zu einer Auflösung des Wirkstoffes, wobei die Blutproteine oft zusätzlich lösungsvermittelnde Wirkung haben.

3. Über Formulierung als Nanosuspension kann eine Reduzierung des Injektionsvolumens von Arzneistoffen erzielt werden. Bei gerinder Wasserlöslichkeit resultiert ein relativ großes zu applizierendes Volumen bei Verabreichung eines Wirkstoffes als Lösung. Alternativ kann der Wirkstoff als Nanosuspension formuliert werden, wobei die Arzneistoffpartikel in einer gesättigten Lösung des Wirkstoffes dispergiert sind. So könnte eins Infusion durch eine Bolusinjektion ersetzt werden.

4. Nanosuspensionen können zur kontrollierten Arzneistoffapplikation eingesetzt werden. Nach oraler Gabe könnte über die M Zellen im Gastrointestinaltrakt eine orale Immunisierung erfolgen, über Bioadhäsiva könnte eine selektive Anreicherung in Absorptionsfenstern des Gastrointestinaltraktes erzielt werden.

5. Nanosuspensionen sind Delivery Systeme für das Drug Targeting. Nach intravenöser Injektion reichern sich Partikel in Abhängigkeit von ihren Oberflächeneigenschaften in bestimmten Organen gezielt an, z.B. Leber, Milz oder Knochenmark (R.H. Müller, Colloidal Carriers for Controlled Drug Delivery and Targeting, Wissenschaftliche Verlagsgesellschaft Stuttgart, 1991). Nach parenteraler Applikation läßt sich eine Anreicherung im Lymphsystem erzielen. Gezielte Anreicherung des Arzneistoffes am Wirkort reduziert die Nebenwirkungen, erhöht die therapeutische Effizienz und damit den therapeutischen Index.

3. Kenntnisstand über Nanosuspensionen und Herstellungstechnologie

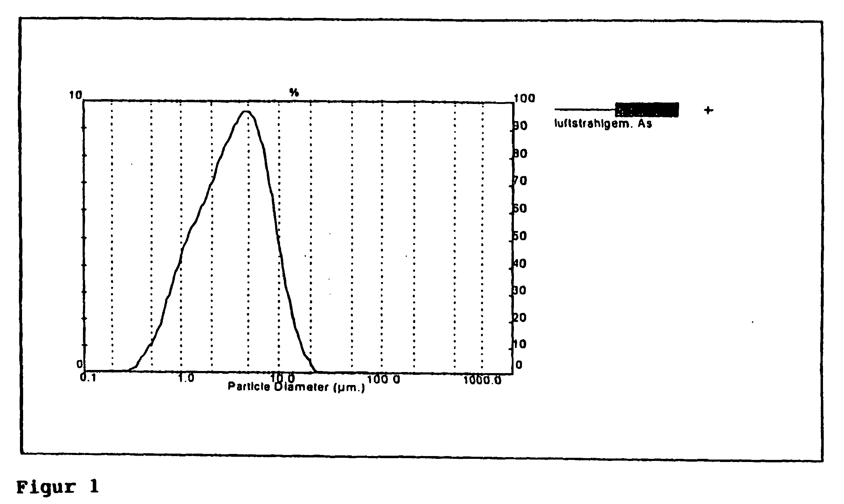

Die Vorteile von Nanosuspensionen konnten bisher nicht ausgenutzt werden, da mit konventionellen Mahltechniken (Trockenmahlung in Kugelmühle, Luftstrahlmahlung) dieser Teilchengrößenbereich nur sehr beschränkt zugänglich ist. Man erhält bei der Luftstrahlmahlung zwar Pulver mit 100% der Teilchen kleiner als ca. 25 - 50 $\mu m$, diese Pulver enthalten jedoch nur einen Anteil von wenigen Prozent an Partikeln im Nanometerbereich. Beispielhaft ist die mit dem Laser Diffractometer (LD) gemessene Teilchengrößenverteilung des luftstrahlgemahlenen Arzneistoffs RMKP 22 (4-[N-(2-Hydroxy-2-methyl-propyl)-ethanolamin]-2,7-bis(cis-2,6-dimethylmorpholin-4-yl)-5-phenyl-pteridin) in Figur 1 dargestellt. 100% der Teilchen sind zwar kleiner als 25 $\mu m$, jedoch nur 8% der Partikel befinden sich in dem Bereich unterhalb 1000 nm, d.h. 92% sind > 1$\mu m$. Man könnte nun annehmen, daß man die Nanometerfraktion abtrennt und die restlichen Partikel einem erneuten Mahlprozeß unterwirft, um so zu weiteren Nanopartikeln zu kommen. Dies ist jedoch nur beschränkt möglich, da man im fortschreitenden Mahlprozeß mit zunehmendem Zerkleinerungsgrad zu immer perfekteren Kristallen kommt, die nachher durch die maximal erreichbaren Mahlkräfte nicht mehr weiter zu zerkleinern sind (P. List, Arzneiformenlehre, Wissenschaftliche Verlagsgesellschaft Stuttgart, 1976). Zusammenfassend ist somit festzustellen, daß Nanopartikel aus Arzneistoffen mit konventineller Trockenmahltechnik und anschließender Fraktionierung hergestellt werden können, jedoch mit einen großen Nachteil: Wirkstoffverlust von ca. mehr als 90%. Die Wirtschaftlichkeit ist in der Regel nicht mehr gegeben.

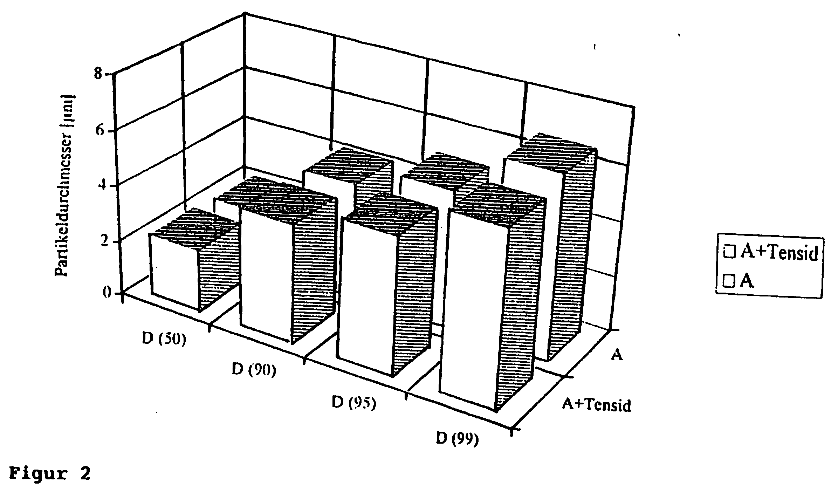

Als weitere Mahltechnik wurde die Naßmahlung eingesetzt (Sandell, E., Grundriss der Galenischen Pharmazie, Govi-Verlag GmbH, Frankfurt am Main, 1962), zum Beispiel unter Verwendung einer Premier Mill Mühle (Sandell, a.a.O.) oder einer Kugel- bzw. Perlmühle (Hagers Handbuch der pharmazeutischen Praxis, Springer - Verlag, Berlin, 1925). Bei Anwendung einer Perlmühle ergibt sich zwar eine Hauptpopulation an Partikeln im Nanometerbereich, jedoch ist noch ein deutlicher Anteil an Partikeln oberhalb von 1 $\mu m$ vorhanden. Figur 2 zeigt die LD- Durchmesser 50%, 90%, 95%, 99% aus der Partikelgrößenverteilung des Arzneistoffes RMKP 22. RMKP 22 wurde (Dispermat) ohne Tensidzusatz (Figur 2: A) und unter Zusatz von 3% Tween 80 (Figur 2: A÷Tensid) in der Perlmühle gemahlen. Es liegt bereits der Durchmesser 50% der tensidfreien Probe bei ca. 2 $\mu m$, d. h. 50% der Partikel sind > als 2 $\mu m$. Ein Teil dieser Mikrometerpartikel kann auf Agglomeration zurückgeführt werden. Wie in der Literatur beschrieben (Sandell, a.a.O.; P.H. List, Arzneiformenlehre, Wissenschaftliche Verlagsgesellschaft mbH Stuttgart, 1976; Sucker, H, Speiser, P., Fuchs, P., Pharmazeutische Technologie, George Thieme Verlag Stuttgart, 1978; Münzel, K., Büchi, J., Schultz, O. -E., Galenisches Praktikum, Wissenschaftliche Verlagsgesellschaft mbH Stuttgart, 1959) kann die Aggregation in Suspensionen durch Zusatz von Tensiden (Tween 80, Pluronic F 68) oder allgemein Stabilisatoren (z.B. Polyvinylpyrrolidon - PVP, Pluronic F 68) verhindert werden. Nach Zusatz von Tween 80 zur Verhinderung der Aggregation ergab sich nur eine geringfügige Reduzierung in den Durchmessern der Volumenverteilung, was den weniger effektiven Verkleinerungsprozeß einer Perlmühle an sich belegt (Figur 2). Eine weitere Reduzierung in der Teilchengröße in derartigen Mühlen ist möglich, wenn die Viskosität des Dispersionsmediums erhöht wird, wobei die Drehzahl jedoch konstant bleiben muß (W. Holley, Dissertation, Friedrichs Universität Karlsruhe, 1984; W. Holley, Homogenisieren mit Hochdruck, Niederdruck, Ultraschall und anderen Techniken, Vortrag 35. Jahreskongreß der APV, Straßburg, 1989). In der Regel wird dies auch von den Mühlenherstellern empfohlen (z.B. Dyno-Mill, A. Bachoffen AG Maschinenfabrik). Tensidstabilisierte Mikropartikel wurden ebenfalls patentiert (United States Patent No. 5,246,707), wobei diese auch noch Eisenpartikel innerhalb der Mikropartikel enthalten können, um eine Lokalisierung der Partikel über magnetische Felder zu ermöglichen.

Die Herstellung von Nanosuspensionen durch Naßmahlung wurde von Motoyama et al. als Verfahren patentiert (U.S. Patent No. 4,540,602)und von Liversidge et al. die Naßmahlung mit einer Perlmühle unter Zusatz von Substanzen wie PVP und Pluronic F68 (U.S. Patent No. 5,145,684). Die Verfahren haben jedoch folgende Nachteile:

1. Es ist nur eine chargenweise Produktion mit einer für industrielle Fertigung zu geringen Ansatzgröße möglich.

2. Es kommt zum Abrieb an den eingesetzten Mahlperlen (Zirkondioxid, Glas). Zirkondioxid und Glasabrieb mag für orale Applikation noch tolerabel erscheinen, jedoch weniger für parenterale oder gar intravenöse Gabe.

3. Es ist immer noch ein relativ großer Anteil an Partikeln > 5 $\mu m$ vorhanden. Analyse der Charge aus Figur 2 mit dem Coulter counter Multisizer II, der sensitiver als ein Laser Diffractometer ist, ergab eine Zahl von 52.671.000 Partikel pro ml einer 5%igen Arzneistoffsuspension, die oberhalb von 5 $\mu m$ waren.

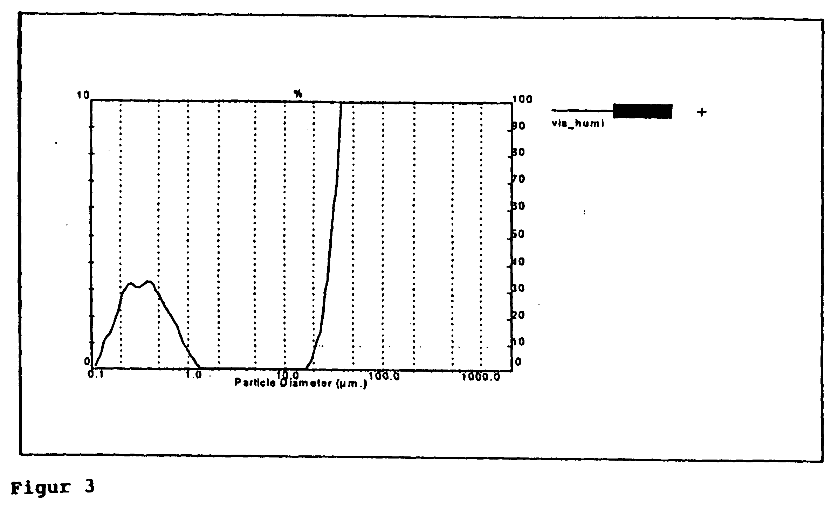

Eine andere seit langem bekannte Herstellungsmethode ist "via humida paratum", die Präzipitation durch Eingießen einer Wirkstofflösung in ein Nichtlösungsmittel (Hagers Handbuch der pharmazeutischen Praxis, Springer -Verlag, Berlin, 1925). Durch Eingießen in das Nichtlösungsmittel wird der Ostwald Miersche Bereich schnell durchlaufen und es kommt zur Ausfällung eines sehr feinen Präzipitates. Die ausgefällten Partikel weisen ebenfalls einen deutlichen Anteil im Mikometerbereich auf. Figur 3 zeigt die Teilchengrößenverteilung (Laser Diffractometer, Volumenverteilung) einer RMKP 22 Suspension, via humida paratum hergestellt. Der Arzneistoff wurde in Ethanol gelöst (3%, 20 ml) und in 100 ml einer wäßrigen Tensidlösung gegossen (1,2% Pluronic F 68). Das Ende des Meßbereiches beträgt 80μm, große Fraktion zwischen ca. 18 μm bis 80 μm wurden detektiert.

Die Herstellung von Nanosuspensionen durch Präzipitation wurde ebenfalls patentiert (EP O 275,796 and EP O 418 151 A1 (Modellarzneistoff: Amphotericin)).

Die Präzipitation hat jedoch folgende <u>Nachteile</u>:

1. Restgehalt des Produktes an Lösungsmitteln, der nur sehr schwer oder nicht vollständig zu entfernen ist.

2. Beim Ausfällen kommt es zu einer Verzögerung der Arzneistoffkristallisation.

3. Teilweise recht hoher Anteil an Partikeln im Mikrometerbereich.

Nanosuspensionen können ebenfalls durch starke Scherkräfte in Flüssigkeiten (jet stream) verbundem mit dem Aufeinanderprallen von Partikeln hergestellt werden: Geräte zur Erzeugung von Flüssigkeitsströmen mit hoher Geschwindigkeit (z.B. 700 m/s) sind der Mikrofluidizer (Microfluidics Inc.) oder der Nanojet (Nanojet Engineering GmbH), eine Weiterentwicklung des Microfluidizers.

WO-A-9 414 426 beschreibt die Herstellung sogenannter mikrofluidisierter Teilchen von Atovaquone in einem Mikrofluidizer. Gemäss der Offenbarung sollen geeigneterweise mindestens 90% der Teilchen einen Durchmesser im Bereich von 0,1-3 μm und vorzugsweise mindestens 95% der Teilchen einen Durchmesser im Bereich von 0,1-2 μm aufweisen. Im Beispiel wurde für ein Gemisch von 2,5% (Gew./Vol.) Atovaquone und 0,25% (Gew./Vol.) Celacol M2500 in Wasser nach 244 Zyklen ein Produkt erhalten, das aufgrund mikroskopischer Beobachtungen als monodispers, bestehend aus sehr kleinen runden Teilchen kleiner als 2,5 μm beschrieben wird.

WO-A-9 006 746 offenbart mittels eines Mikrofluidizers hergestellte Öl-in-Wasser-Emulsionen, in denen die Tröpfchen der Ölphase einen mittleren Durchmesser von weniger als etwa 0,4 μm aufweisen und die dehydratisierbar und anschliessend durch Zugabe von Wasser wieder in Emulsionen mit ähnlicher Tröpfchengrösse überführbar sind. Zur Herstellung der Emulsion wurde eine Kombination von etwa 0,1-60 Gew.-% eines lipidlöslichen Materials, etwa 0,1-10 Gew.-% eines Öl-in-Wasser-Emulgators, etwa 0,5-70 Gew.-% eines hydrophilen wasserlöslichen festen Kohlehydrats und Wasser verwendet.

Aus EP-A-0 361 928 sind Emulsionen mit einer mittleren Tröpfchengrösse von 0,010-0,070 μm bekannt, die mittels Hochdruckhomogenisation mit einem Mikrofluidizers erhalten wurden. Die offenbarten Emulsionen umfassen (A) einen lipidlöslichen Wirkstoff und ein Lipid, (B) Glycerin und Wasser und (C) ein Phospholipid und/oder ein wasserlösliches nicht-ionisches oberflächenaktives Mittel mit einem Molekulargewicht von 1000 oder mehr, wobei das Gewichtsverhältnis (A)/(C) 0,5 bis 5 beträgt.

WO-A-9 318 752 beschreibt Zusammensetzungen zur topischen Anwendung von Pharmazeutika oder Kosmetika mit erhöhter topischer und/oder transdermaler systemischer Wirkung, umfassend Tröpfchen eines Wirkstoffes und öliger Exzipientien mit einer Tröpfchengrösse von vorzugsweise etwa 0,05-0,5 μm, allein oder dispergiert in einem wässrigen Medium. Die Tröpfchen enthalten etwa 0,5-30% einer öligen Flüssigkeit, etwa 0,1-10% eines Emulgators und etwa 0,05-5% eines nichtionischen oberflächenaktiven Mittels. Der Wirkstoff ist entweder selbst eine ölige Flüssigkeit oder in einer öligen Flüssigkeit gelöst oder dispergiert. Die gewünschten Tröpfchengrösse wird durch intensives Mischen, notfalls unter Einsatz eines Mischers mit hohen Scherkräften, eines Hochdruckhomogenisators oder durch Beschallung, erreicht.

Aus DE-A-4 217 842 sind wässrige, öl und Calciumantagoni-. sten enthaltende Emulsionen zur intravenösen und/oder intrakoronaren Anwendung bekannt, die durch Lösen einer entsprechenden Menge der Calciumantagonisten in einem geeigneten Öl, Emulgieren dieser öligen Lösung in Wasser bei hohen Drücken in einem Hochdruck-Homogenisator unter Verwendung von 3-sn-Phosphatidylcholin und gegebenenfalls einem Alkalisalz einer freien Fettsäure als Emulgator und Sterilisation der erhaltenen Emulsion bei hohen Temperaturen hergestellt werden. Die in den Beispielen beschriebenen Emulsionen enthalten keine oder weniger als 1% an Tröpfchen mit einer Grösse über 3,2 μm und die Mehrzahl der Teilchen liegt jeweils im Submicron-Bereich.

In Eur. J. Pharm. 40 (3), 157-160 (1994) wurden zur Untersuchung der parenteralen Verabreichbarkeit von Fettemulsionen Emulsionen, bestehend aus 10% Sojaöl, 1,2% Eilecithin und Wasser, hergestellt und mittels eines Hochdruckhomogenisators (Micron Lab 40, APV Gaulin) homogenisiert. Die Tröpfchendurchmesser D(50%) lagen jeweils über 0,5 μm und D(95%) über 1 μm. Die Autoren kommen zum Schluss, dass Emulsionen entweder bei niedrigem bis mittlerem Druck und hoher Temperatur oder bei mittlerem bis hohem Druck und tiefer Temperatur hergestellt werden sollten.

Aus WO-A-9 420 072 sind ferner Suspensionen kolloidaler fester Lipid-Teilchen (solid lipid particles, SLPs) und Suspensionen von Teilchen biologisch wirksamer Mittel (particles of bioactive agents, PBAs) im Mikron- und Submikron-. Bereich bekannt, die durch Abkühlen entsprechender Emulsionen erhalten werden. Die SLPs können in der Lipidmatrix Wirkstoffe enthalten und damit als Träger zur kontrollierten Freisetzung von schlecht wasserlöslichen Substanzen dienen. Die Matrix der SLPs besteht aus biokompatiblen, hydrophoben, bei Raumtemperatur festen Materialien mit Schmelzpunkten im Bereich von etwa 30-120°C. Zur Herstellung der SLPs werden (1) das Lipid oder die Lipidmischung geschmolzen, (2) die Stabilisatoren zum Lipid und/oder zum Dispersionsmedium zugesetzt, (3) allfällige Wirkstoffe zusammen mit den Lipiden geschmolzen oder in der Lipidschmelze gelöst, solubilisiert oder dispergiert, (4) das Dispersionsmedium auf die Temperatur der Schmelze erhitzt, (5) die geschmolzenen Lipide im Dispersionsmedium emulgiert, was vorzugsweise durch Hochdruckhomogenisation erfolgen kann, (6) die Dispersion sterilisiert, (7) die Dispersion unter Kristallisation der dispergierten Lipide und Bildung von SLPs abkühlen gelassen, (8) das Volumen des Dispersionsmediums verringert. In ähnlicher Weise erfolgt auch die Herstellung der PBAs, was voraussetzt, dass der Wirkstoff selbst im Temperaturbereich von etwa 30-120°C schmelzbar ist. Im Falle von PBAs wurden mittels Hochdruckhomogenisation mit einem APV Gaulin Micron Lab 40 mittlere Teilchengrössen von 21,8 µm für Miconazole, 61,4 µm für Ibuprofen, 174,2 µm für Lidocaine und 325,1 nm für Cholecalciferol, d.h. nur ausnahmsweise mittlere Teilchengrössen im Submikron-Bereich erreicht.

3. Beschreibung der Erfindung:

Die Erfindung betrifft ein Verfahren gemäss Patentanspruch 1. Bevorzugte Ausführungsformen sind in den abhängigen Patentansprüchen 2 bis 38 definiert.

Hauptschwierigkeiten bei der Herstellung von Nanosuspensionen sind u.a. die Reduzierung des Anteils an Partikeln im Mikrometerbereich (besonders an Partikeln grösser als 5 µm bei Suspensionen zur i.v. Applikation) sowie die Verwendung eines Verfahrens, das sowohl die grosstechnische Produktion erlaubt als auch gleichzeitig ein Produkt ergibt, das aus toxikologischer Sicht als Arzneimittel durch die Zulassungsbehörden (Bundesgesundheitsamt in der BRD, FDA in den USA) zulassungsfähig ist. Zur Dispergierung von Ölen im Rahmen der Produktion von Fettemulsionen zur parenteralen Ernährung werden zur grosstechnischen Produktion seit vielen Jahren Kolben-Spalt-Hochdruckhomogenisatoren eingesetzt. Das Dispergierungsprinzip ist die Kavitation. Hierbei wird eine grobdisperse Präemulsion durch einen ca. 25 µm breiten Spalt gedrückt. Hierbei sinkt nach Bernoulli (Sucker, H., Speiser, P., Fuchs, P., Pharmazeutische Technologie, George Thieme Verlag Stuttgart, 1978) aufgrund der hohen Strömungsgeschwindigkeit der auf der Flüssigkeit lastende statische Druck unterhalb des Dampfdruckes der Flüssigkeit bei der herrschenden Temperatur ab. Die Flüssigkeit siedet, es kommt zur Bildung von Gasblasen, die beim Austritt aus dem Spalt unter nun herrschendem Normaldruck kollabieren (Kavitation). Durch die starken Implosionskräfte kommt es zum Zerreissen der Öltröpfen in Tropfen einer Grösse von ca. 200 bis 500 nm. Eine Eignung dieses Dispergierungssystems zum Zerkleinern von Feststoffen - zugeführt in Form einer groben Suspension - wurde als nicht gegeben betrachtet, da erwartet wurde, das der Spalt durch Pulverpartikel mit einer Grösse von bis zu 50 µm oder auch durch Aggregation kleinerer Partikel verstopfen würde. Auch war fraglich, ob die Implosionskräfte zur Zerkleinerung von Kristallen mit wenig Fehlstellen, d.h. sehr harten Kristallen, ausreichten.

Es wurden Suspensionen mit luftstrahlgemahlenem Arzneistoff in wäßriger Tensidlösung hergestellt. Die Arzneistoffkonzentration betrug 3%, 9% und 15%. Als Modellarzneistoff wurde RMKP 22 eingesetzt. Die Suspension wurde im Kolben-Spalt-Homogenisator unter den Bedingungen 1500 bar, drei Zyklen homogenisiert. Die Durchmesser der resultierenden Nanopartikel sanken vom 1. bis zum 3. Zyklus (Beispiele 1 bis 3).

Es wurde der Durchmesser der Hauptpopulation und der Anteil an Partikeln im Mikrometerbereich als Funktion der Zyklenzahl untersucht. Der Durchmesser der Hauptpopulation und der Anteil an Partikeln im Mikrometerbereich nahmen mit der Anzahl der Zyklen ab, wobei ein starker Abfall während der ersten 3 bzw. 6 Zyklen auftrat, ein leichterer Abfall vom 5. bzw. 7. bis zum 10. Zyklus, keine Änderung mehr ab dem 10. Zyklus aufgrund des Erreichens der Grenzdispersität unter der bei 1500 bar sich ergebenden Leistungsdichte (Beispiele 4 und 5).

Nanosuspensionen, die nach 10 Zyklen erhalten wurden, zeigten einen mehrfach geringeren Anteil an Partikeln > 1 µm und > 5 µm pro Volumeneinheit als kommerzielle Fettemulsionen zur parenteralen Ernährung (Beispiel 6). Bei Fettemulsionen auftretende Kapillarblockade ist durch die Metabolisierung der Fettemulsion reversibel. In ca. 4 Stunden wird eine applizierte Fettemulsion von den endothelständigen Lipasen abgebaut. Bei der Nanosuspension ist eine Blockade durch die Auflösung des Nanoteilchens reversibel. Aufgrund der erhöhten Sättigungslöslichkeit (Beispiel 7) kommt es zu einem schnellen Auflösungsprozeß der Nanoteilchen bei Verdünnung der Nanosuspension (Beispiel 8).

Durch Verkleinern des Durchmessers der Mikropartikel von 3,54 µm (Dm) auf den Durchmesser der Nanosuspension von 800 nm (Dn) kam es zu einer starken Erhöhung der Sättigungslöslichkeit. Durch Schüttelexperimente wurde eine Sättigungskonzentration für die RMKP 22 - Mikropartikelsuspension von Csm 1,98 mg/l, für die RMKP 22 - Nanosuspension eine deutlich höher Csn von 3,29 mg/l ermittelt (Beispiel 7).

Durch Verkleinerung der Teilchengröße wurde eine Erhöhung der Sättigungslöslichkeit zwar erwartet, jedoch nicht in dieser Höhe. Eine Erhöhung der Sättigungslöslichkeit bei Verkleinerung der Teilchengröße wird in der Gleichung von Ostwald-Freundlich postuliert (Voigt, R., Lehrbuch der pharmazeutischen Technologie, Verlag Chemie Berlin, 1984), wobei diese jedoch bei Partikeln im Bereich von Mikrometern nicht zum Tragen kommt (alleinige Abhängigkeit der Sättigungslöslichkeit als substanzspezifische Kenngröße von der Temperatur):

$$\frac{R\,T}{M} \cdot \ln \frac{Csm}{Csn} = \frac{4y}{\sigma}\ (1/Dn - 1/Dm)$$

| R | - universelle Gaskonstante | T | - absolute Temperatur |
|---|---|---|---|
| M | - Molekülmasse | | |
| Dm | - Durchmesser Mikropartikel | Csm | - Sättigungslöslichkeit Mikropartikel |
| Dn | - Durchmesser Nanopartikel | Csn | - Sättigungslöslichkeit Nanosuspension |
| y | - Grenzflächenspannung Wirkstoff | o | - Dichte |

Die in dieser Höhe aufgetretene Steigerung der Sättigungslöslichkeit ist schwerlich mit der relativ geringen Teilchengrößendifferenz zu erklären. Der einzige mögliche variable Parameter in obiger Gleichung ist die Grenzflächenspannung y. Nach Ostwald-Freundlich ist die beobachtete Erhöhung der Sättigungslöslichkeit nur erklärbar durch eine nicht erwartete Änderung der Grenzflächenspannung y, die durch den Homogenisationsprozeß erfolgt sein muß. Der Energieeintrag während des Homogenisationsprozesses muß zu einer Erhöhung von y und einer damit verbundenen Erhöhung von der Sättigungslöslichkeit geführt haben. Somit ist es offensichtlich möglich, durch Überführung der Mikropartikel in Nanopartikel mittels eines hochenergetischen Prozesses die Grenzflächenspannung so stark zu erhöhen, daß daraus resultierend die Sättigungslöslichkeit stark ansteigt.

Polymorphie, als eine mögliche Ursache für die höhere Sättigungslöslichkeit, konnte nicht nachgewiesen werden. Im Röntgendiffraktogramm ergeben sich keine Unterschiede zwischen Mikropartikeln und der Nanosuspension. Eine andere mögliche Ursache ist die Hydrophobisierung der Oberfläche durch Aufbrechen von "idealen" Kristallen, die mit konventioneller Mahltechnik nicht zerstörbar sind. Bruchflächen entstehen nicht mehr bevorzugt an Fehlstellen (List, a. a.O.), sondern gehen direkt durch das Kristall. Besitzen die aus einem idealen Kristall neu entstandenen Bruchflächen eine höhere Grenzflächenspannung, so resultiert daraus eine höhere Sättigungslöslichkeit. Ein weiterer möglicher Effekt, der nicht ausgeschlossen wird, ist die Veränderung des Krümmungsradius. Die Packungsdichte der Tenside an der Oberfläche ist durch die geänderten geometrischen Verhältnisse nicht mehr optimal, daß heißt weniger dicht gepackt. Daraus resultiert eine erhöhte Grenzflächenspannung an der Grenzfläche der Nanopartikel.

Aus bisherigen Lagerdaten über mehrere Wochen ist dieser Zustand einer höher gesättigten Lösung auch stabil, Partikelwachstum durch Rekristallisation fand nicht statt. Die Noyes - Whitney Gleichung beschreibt die Lösungsgeschwindigkeit dc/dt (Stricker, H. (Hrsg), Physikalische Pharmazie, Wissenschaftliche Verlagsgesellschaft, Stuttgart 1987):

$$\frac{dc}{dt} = DA \cdot \frac{(Cs - Ct)}{dx}$$

| dc/dt. | - Lösungsgeschwindigkeit | D | - Diffusionskonstante |
|---|---|---|---|
| A | - Oberfläche der Partikel | Cs | - Sättigungskonzentration |
| Ct | - Konzentration zur Zeit t im Lösungsmedium | | |
| dx | - Distanz zwischen gesärtigter Schicht an der Teilchenoberfläche und dem Ort mit Ct | | |

Von den theoretischen Überlegungen war durch die Überführung der Mikropartikel in die Nanopartikel eine Steigerung der Lösungsgeschwindigkeit lediglich aufgrund der Vergrößerung der Oberfläche A erwartet worden, was bei Überführung eines 3,64 μm Partikels in ein 800 nm Teilchen eine Steigerung um den Faktor 4,55 ausmacht. Durch die überraschenderweise aufgetretene Erhöhung von Sättigungslöslichkeit (aufgrund der unerwarteten deutlichen Änderung der Grenzflächenspannung y) kommt es zu einer zusätzlichen Erhöhung der Lösungsgeschwindigkeit. Dies bewirkt selbst in Lösungen mit einer Konzentration von Csm eine schnelle Partikelauflösung (Beispiel 8). Für eine intravenöse Applikation von Nanosuspensionen hat dies natürlich den Vorteil, daß es aufgrund der großen Verdünnung (z.B. 10 ml in 6 l) im Blut zu einer raschen Auflösung der injizierten Substanz kommt. Weiter fördernd auf die Auflösung wirken die in Blut vorhandenen Proteine über eine mögliche Solubilisierung von Wirkstoffen.

Als hochdisperses System kann bei den Nanosuspensionen eine Instabilität während der Lagerung nicht ausgeschlossen werden. Die Langzeitstabilität wurde daher mit PCS und Laser Diffractometrie untersucht. In Nanosuspensionen mit optimierter Zusammensetzung wurde über 8 Wochen Lagerung bei 4 - 8° C kein Teilchenwachstum detektiert (Beispiel 9).

Es wurden Untersuchungen zur Sterilisierbarkeit mittels Autoklavieren und auch mittels Gamma- durchgeführt. Der Einfluß folgender Parameter auf die Sterilisierbarkeit wurde bestimmt:

a. die chemische Natur des Tensides (z.B. Lecithine, verschiedene Phospholipide sowie als ethoxylierte Stabilisatoren Tween 80 und Pluronic)

b. Mischungen aus zwei und mehr Tensiden

c. die Konzentration der Tenside oder Stabilisatoren.

Aufgrund theoretischer Überlegungen sollten die Tensid- oder Stabilisatorkonzentration deutlich oberhalb der Konzentration zur Erreichung des Plateaus der Adsorptionsisothermen liegen, damit die Oberflächen der dispergierten Partikel dicht mit stabilisierenden Molekülen bedeckt sind. Bei ungenügender Oberflächenbelegung kann es zur Aggregatbildung mittels eines Bridging, anchoring oder einer Flockung durch Wechselwirkung hydrophober Tensidteile kommen (B. W. Müller, P. List, Arzneiformenlehre, im Druck). Speziell für sterische Stabilisatoren (z.B. ethoxylierte Blockcopolymere wie Pluronic) ist die Überschreitung der Plateaukonzentration wichtig, da damit die maximale Adsorptionsschichtdicke erreicht wird (Kayes, J.B. and Rawlins, D.A. , Adsorption charakteristics of certain polyoxyethylene-polyoxypropylene block copolymers on polystyrene latex, Coll. Polym. Sci. 1979, 257, 622-629). Die sterische Stabilisierung nimmt mit der Schichtdicke zu, wobei zur perfekten sterischen Stabilisierung eine Schichtdicke von >10 nm erforderlich ist (Buscall, R. and Ottewill, R.H., The stability of polymer latices in Polymer Colloids, Elsevier Applied Science Publishers, London, 1986, pp 141-217). Oft ist es vorteilhaft, stark über die Plateaukonzentration hinauszugehen, da dann eine Stabilisierung durch Verdrängung möglich ist (B. W. Müller, P. List, Arzneiformenlehre, im Druck). Beim Annähern zweier Partikel werden die Tenside aus dem Zwischenraum verdrängt, es entsteht eine tensidfreie Zone zwischen den Partikeln. Zwischen der tensidfreien Zone und der umgebenden Tensidlösung besteht nun eine osmotische Druckdifferenz, Tensidmoleküle drängen aufgrund dieser Differenz zwischen die Partikel, schieben sie wieder auseinander und verhindern so die Aggregation. Das Hineinschieben ist um so ausgeprägter, je größer die osmotische Differenz ist, das heißt je höher die Tensidkonzentration in der Dispersion ist. Aufgrund oben ausgeführter Überlegungen kommen daher Tensidkonzentrationen im Bereich von einem bis mehreren Prozent zum Einsatz. Die Standardtensidkonzentration in O/W Emulsionen zur parenteralen Ernährung ist daher auch 1,2% Lecithin (z.B. Handelsprodukte wie Intralipid, Lipofundin, Endolipide, Lipovenös etc.). Höhere Konzentrationen werden in der Literatur auch als wesentlich stabilisierender als 0.6% beschrieben und auch verwendet (Meyer, C.E., Fander, J.A., Schurr, P. E., Webster, H.D., Metabolismo 5, 591, 1957). Für ethoxylierte Tenside vom Typ Pluronic (Poloxamer) werden zur Erreichung des Plateaus der Adsorptionsisothermen ebenfalls - in Abhängigkeit vom Poloxamer-Typ, Werte im Bereich von 0.05% bis 0,1% angegeben (Kayes and Rawlins, a.a.O.; Wesemeyer, H., Dissertation, Christian - Albrechts - Universität Kiel, 1993), so daß hier ebenfalls zur Stabilisierung in der Regel Konzentrationen ab 1% aufwärts eingesetzt werden, oft sogar zuzüglich eines oder mehrerer anderer Kotenside, was insgesamt zu Tensidkonzentrationen bis 5% führt (Schwarz, C., Mehnert, W., Lucks, J.S., Müller, R.H., Solid lipid nanoparticles for controlled drug delivery, Journal of controlled release, 1994; Westesen, K., Siekmann, B., Submicron-sized parenteral carrier systems based on solid lipids, Pharmaceutical and Pharmacological Letters, Springer-Verlag 1992).

Die Sterilisierung von Nanosuspensionen stabilisiert mit unterschiedlichen Tensidkonzentratron ergab jedoch überraschenderweise geringstes Teilchenwachstum bei einer Tween 80 - Konzentration von 0.03% bis 0.1%, das heißt im Bereich der Konzentration zur Erreichung des Plateaus der Adsorptionsisothermen bzw. auch leicht darunter (Beispiel 12). Dies bedeutet, daß Nanosuspensionen bei sehr geringen Tensid- und Stabilisatorkonzentrationen optimale Ausgangssuspensionen für das Autoklavieren darstellen.

Da aus toxikologischer Sicht ein möglichst geringer Tensidgehalt wünschenswert ist, wurden auch tensidfreie Nanosuspensionen hergestellt (Beispiel 13). Eingesetzter Wirkstoff war Carbamazepin, zur Verringerung der Sedimentation beim Durchpumpen durch den Homogenisator wurde zur Viskositätserhöhung Natriumcarboxymethylcellulose zugesetzt.

Der jet stream wurde als Dispergierprinzip ebenfalls auf seine Eignung untersucht. Es wurden ebenfalls qualitativ hochwertige Nanosuspensionen erhalten (Vergleichsbeispiel 14). Nachteilig bei diesem Prinzip ist seine bisherige noch relativ geringe Verbreitung in den Produktionsanlagen der pharmazeutischen Industrie.

Die bei der Dispergierung erhaltene Teilchengröße ist eine Funktion der eingesetzten Leistungsdichte, der Härte des Arzneistoffes, der Viskosität des Dispersionsmediums (Anstieg der Leistungsdichte mit der Viskosität bei gleichbleibender Strömungsgeschwindigkeit der dispergierenden Phase) sowie der Tensideigenschaften (z.B. Wanderungsgeschwindigkeit der Tenside auf im Dispergierprozeß neu gebildete Oberflächen, stabilisierende Wirkung des Tensides auf der Oberfläche im Dispergierprozeß, d.h. unter Streßbelastung der Suspension aufgrund des hohen Eintrages an kinetischer Energie). Durch Modifikation der Herstellungsparameter einerseits und der Rezepturzusammensetzung andererseits kann die erhaltene Teilchengröße beeinflußt werden. Herstellurgsperameter und Rezepturzusammensetzung für eine Nanosuspension mit sehr kleinem mittleren Durchmesser gibt Beispiel 15.

Die Anwendungsgebiete für die erfindungsgemäß erhältlichen Arzneistoffträger sind vielfältig. Beispielsweise können sie zu parenteralen (besonders intravenösen Applikation und zur lymphatischen Absorption), enteralen (besonders mucoadhäsive Arzneiformen), pulmonalen und topischen (nasal, dermal, intraoculär) Arzneistoffapplikation und zur Applikation in Körperhöhlen verwendet werden.

Bei der parenteralen Applikation handelt es sich um:

1. Intravenöse Gabe (Targeting zu Leber, Milz, Knochenmark, Lunge, Blutzellen wie Lymphozyten, Monozyten und Granulozyten, Erzeugung von im Blut zirkulierenden Teilchen mit kontinuierlicher Auflösung des Wirkstoffes im Kompartiment Blut).

2. Lymphatische Aufnahme von Arzneistoffträgern durch Injektion in der Nähe von Lymphgefäßen (Targeting von Cytostatika zu Lymphknoten)

3. Intramuskuläre Gabe (Depotform für verlängerte oder langanhaltende Freisetzung von Wirkstoffen, z.B. Kortikoide. Aufgrund der reduzierten Flüssigkeitsmenge im Gewebe kommt es zu einem verlangsamten Auflöseprozeß, vor allem von schwer- bis praktisch unlöslichen Wirkstoffen).

4. Intraartikuläre Gabe (z.B. für Antirheumatika und Immunsuppressiva bei Arthritis).

5. Intrakavitale Gabe (z.B. Cytostatika für Krebsformen im Peritoneum und in der Pleurahöhle)

6. Subkutane und intradermale Gabe (z.B. Depotformen für Cytostatika bei Hautkrebs)

Die enteralen Applikationsformen dienen insbesondere zur:

1. Erhöhung der Resorption durch Herstellung mucoadhäsiver Arzneistoffträger, die vermehrt sich an die Mucosa anlagern und dort auch länger verbleiben.

2. Oralen Immunisierung durch Wechselwirkung der Arzneistoffträger mit z.B. M Zellen in den Peyer's Patches.

3. Aufnahme von Wirkstoffen über die M Zellen.

4. Resorptionserhöhung lipophiler Wirkstoffe durch unspezifische Anlagerung an die Mucosa, z.B. lipophile Vitamine.

5. Aufnahme von Arzneistoffträgern ins lymphatische System.

Als pulmonale Applikationsformen kommen insbesondere in Betracht :

1., Aerosole, Dosieraerosole (Versprühen einer wäßrigen Dispersion der Arzneistoffträger)

2. Aerosole, Dosieraerosole (Versprühen eines Pulvers, wobei die Arzneistoffträger im Nanometerbereich auf Trägerpartikel wie Lactose im Mikrometerbereich aufgesprüht wurden. Die Lactose löst sich in der Lunge und setzt die Arzneistoffträger frei, z.B. zwecks Aufnahme durch Makrophagen oder z.B. sie verbleiben auf der Lungenoberfläche und es lösen sich Wirkstoffe mit Zielgruppe Peritonealzellen I oder II auf.

3. Instillation der Dispersion, wobei hier evtl. die Spreitung fördernde Substanzen wie Phospholipide oder Phospholipid - assoziierte Proteine zugesetzt werden.

Beispiele für die topische Anwendung:

1. Dermatologische Arzneimittel zur Applikation von z.B. Kortikoiden und Antimykotika. Durch die erhöhte Sättigungslöslichkeit der Arzneistoffträger entsteht ein höherer Konzentrationsgradient als bei Wirkstoffkristallen im Mikrometerbereich, die Aufnahme in die Haut ist begünstigt. Zusätzlich besteht bei den Arzneistoffträgern aufgrund ihrer geringen Größe die Möglichkeit, zwischen die Zwischenräume der Stramm corneun Zellen zu gelangen (analog zu Liposomen), was ebenfalls die Hautaufnahme begünstigt.

2. Augensuspensionen, Augengele oder Inserte, z.B. für Pilocarpin oder Betablocker. Aufgrund der partikulären Struktur kommt es zu verlängerten Verweilzeiten wie bereits für Nanopartikel aus Polymeren beschrieben. Die Inserte bewirken aufgrund der langsamen Löslichkeit eine sustained release ohne Verwendung einer Steuermembran.

3. Kosmetika analog den liposomalen Präparaten.

4. Partikuläre Applikation von Wirkstoffen in die Nase zwecks nasaler Resorption.

Beispiele für in Form einer Nanosuspension zu verarbeitende Azneistoffgrupen sind (ggf. in Form ihrer wenig wasserlöslichen Form, z.B. als Base anstelle des Hydrochlorids):

1. Analgetika / Antirheumatika
z.B. Morphin, Codein, Piritramid, Fentanyl, Levo-methadon, Tramadol, Diclofenac, Ibuprofen, Indo-metacin, Naproxen, Piroxicam

2. Antiallergika
z.B. Pheniramin, Dimetinden, Terfenadin, Astemizol, Loratidin, Doxylamin, Meclozin

3. Antibiotika / Chemotherapeutika
z.B. Rifamoicin, Ethambutol, Thiazetazon

4. Antiepileptika
z.B. Carbamazepin, Clonazepam, Mesuximid, Phenytoin, Valproinsäure

5. Antimykotika

a) intern:
z.B. Natamycin, Amphotericin B, Miconazol
b) extern außerdem:
z.B. Clotrimazol, Econazol, Fenticonazol, Bifonazol, Ketoconazol, Tolnaftat

6. Corticoide (Interna)
z.B. Aldosteron, Fludrocortison, Betametason, Dexametason, Triamcinolon, Fluocortolon, Hydroxycortison, Prednisolon, Prednyliden, Cloprednol, Methylprednisolon

7. Dermatika

a) Antibiotika:
z.B. Tetracyclin, Erythromycin, Framycetin, Tyrothricin, Fusidinsäure
b) Virustatika wie oben, außerdem:
z.B. Vidarabin
c) Corticoide wie oben, außerdem:
z.B. Amcinonid, Flupredniden, Alclometason, Clobetasol, Diflorason, Halcinonid, Fluocinolon, Clocortolon, Flumetason, Diflucortolon, Fludroxycortid, Halometason, Desoximetason, Fluocinolid, Fluocortinbutyl, Flupredniden, Prednicarbat, Desonid

10. Hypnotika, Sedativa
z.B. Cyclobarbital, Pentobarbital, Methaqualon, Benzodiazepine (Flurazepam, Midazolam, Nitrazepam, Lormetazepam, Flunitrazepam, Triazolam, Brotizolam, Temazepam, Loprazolam)

12. Immuntherapeutika und Zytokine
z.B. Azathioprin, Ciclosporin

13. Lokalanaesthetika

a) intern:
z.B. Butanilicain, Mepivacain, Bupivacain, Etidocain, Lidocain, Articain
b) extern außerdem:
z.B. Oxybuprocain, Tetracain, Benzocain

14. Migränemittel
z.B. Lisurid, Methysergid, Dihydroergotamin, Ergotamin

15. Narkosemittel
z.B. Methohexital, Propofol, Etomidat, Ketamin, Thiopental, Droperidol, Fentanyl

16. Nebenschilddrüsenhormone, Calciumstoffwechselregulatoren
z.B. Dihydrotachysterol

17. Ophthalmika
z.B. Cyclodrin, Cyclopentolat, Hormatropin, Tropicamid, Pholedrin, Edoxudin, Aciclovir, Acetazolamid, Diclofenamid, Carteolol, Timolol, Metipranolol, Betaxolol, Pindolol, Bupranolol, Levobununol, Carbachol

18. Psychopharmaka
z.B. Benzodiazepine (Lorazepam, Diazepam), Clomethiazol

21. Sexualhormone und Ihre Hemmstoffe
z.B. Anabolika, Androgene, Antiandrogene, Gestagene, Estrogene, Antiestrogene

22. Zytostatika und Metastasehemmer

a) Alkylantien wie Melphalan, Carmustin, Lomustin, Cyclophosphamid, Ifosfamid, Trofosfamid, Chlorambucil, Busulfan, Prednimustin, Thiotepa
b) Antimetabolite wie Fluorouracil, Methotrexat, Mercaptopurin, Tioguanin
c) Alkaloide wie Vinblastin, Vincristin, Vindesin
d) Antibiotoka wie Dactinomycin
e) Taxol und verwandte bzw. analoge Verbindungen
f) Dacarbazin, Estramustin, Etoposid

4. Beispiele:

Die Erfindung wird in den folgenden Beispielen näher erläutert:

Beispiel 1

Herstellung einer 3%igen Nanosuspension mit RMKP 22 (4-[N-(2-hydroxy-2-methyl-propyl)-ethanolamin]-2,7-bis(cis-2,6-dimethylmorpholin-4-yl)-6-phenyl-pteridin):

| Grundrezeptur | RMKP 22 | | 3,0 |
|---|---|---|---|
| | Tween 80 | | 0,1 |
| | Aqua dest. | ad | 100,0 |

Der pulverisierte Arzneistoff (luftstrahlgemahlen, Partikel bis über 25 µm) wurde mit einer konzentrierten Tensidlösung zur Benetzung in einer Reibschale angerieben, dann wit dem restlichen, Aqua dest. unter Reiben aufgefüllt. Alternativ kann das Arzneistoffpulver auch unter Rühren in eine Tensidlösung eingebracht werden. Diese grobdisperse Suspension wurde dann bei Raumtemperatur durch einen kontinuierlich arbeitenden Micron LAB 40 gegeben. Homogenisationsbedingungen: 1500 bar, 1 - 4 Zyklen. Der mittlere Teilchendurchmesser der Stammsuspensionen (= Suspension mit 0 Zyklen) mit dem Laser Diffractometer gemessen, die resultierenden Nanosuspensionen mit PCS (PI = Polydispersitätsindex):

| Durchmesser | PI | |
|---|---|---|
| Suspension mit 0 Zyklus | 3250 nm | |
| Suspension mit 2 Zyklen | 406 nm | 0,244 |
| Suspension mit 4 Zyklen | 208 nm | 0,770 |

| Grundrezeptur | RMKP 22 | | 3,0 |
|---|---|---|---|
| | Tween 80 | | 1,0 |
| | Aqua dest. | ad | 100,0 |

Herstellung wie bei Beispiel 1. Die resultierenden Nanosuspensionen hatten folgende PCS Kenndaten:

| Durchmesser | PI | |
|---|---|---|
| Suspension mit 0 Zyklus | 3250 nm | |
| Suspension mit 2 Zyklen | 345 nm | 0,197 |
| Suspension mit 4 Zyklen | 242 nm | 0,188 |

Beispiel 2

Herstellung einer 9%igen Nanosuspension mit RMKP 22:

| Grundrezeptur | RMKP 22 | | 9,0 |
|---|---|---|---|
| | Tween 80 | | 0,3 |
| | Mannit | | 16,7 |
| | Aqua dest. | ad | 100,0 |

Herstellung wie bei Beispiel 1. Die resultierenden Nanosuspensionen hatten folgende PCS Kenndaten:

| Durchmesser | PI |
|---|---|
| Suspension mit 0 Zyklus: | 3170 nm |
| Suspension mit 1 Zyklen: | 817 nm 0,288 |
| Suspension mit 2 Zyklen: | 914 nm 0,425 |
| Suspension mit 3 Zyklen: | 646 nm 0,395 |
| Suspension mit 4 Zyklen: | 606 nm 0,276 |

Beispiel 3

Herstellung einer 15% igen Nanosuspension mit RMKP:

| Grundrezeptur | RMKP 22 | | 15,0 |
|---|---|---|---|
| | Tween 80 | | 0,5 |
| | Mannit | | 16,7 |
| | Aqua dest. | ad | 100,0 |

Herstellung wie bei Beispiel 1. Die resultierenden Nanosuspensionnen hatten folgende PCS Kenndaten:

| Durchmesser PI | |
| --- | --- |
| Suspension mit 0 Zyklus | 2880 nm |
| Suspension mit 2 Zyklen | 273 nm 0,154 |

Beispiel 4

Herstellung einer 9% igen Nanosuspension mit RMKP 22 / Tween 80: Durchmesser der Nanosuspension als Funktion der Zyklenzahl:

| Grundrezeptur | RMKP 22 | | 9,0 |
| --- | --- | --- | --- |
| | Glycerol 85% | | 16,7 |
| | Tween 80 | | 0,3 |
| | Aqua dest. | ad | 100,0 |

Herstellung der Suspension und anschließende Homogenisation wie in Beispiel 1. Homogenisationsparameter: 1500 bar, 1 bis 7 Zyklen. Die Nanosuspension wurde mit der PCS vermessen.

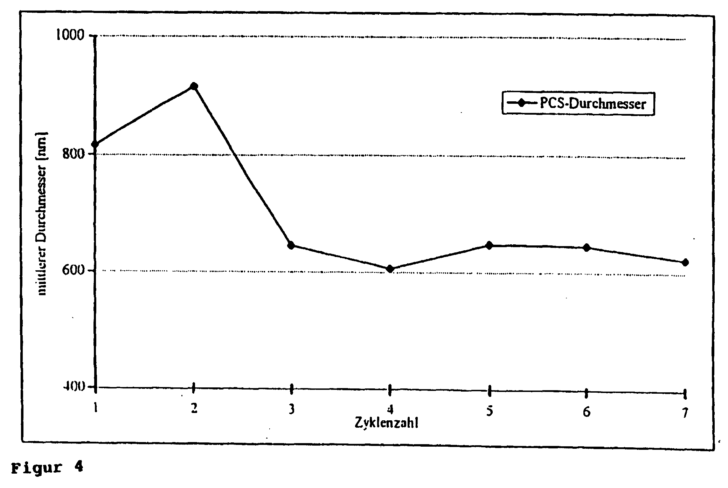

In Figur 4 werden die PCS-Durchmesser als Funktion der Zyklenzahl aufgetragen. Bereits nach ca. 3 Zyklen wird fast der minimale Durchmesser der Nanosuspension von 610 nm erzielt.

Um die Injizierbarkeit von Nanosuspensionen zu beurteilen wurden absolute Partikelzahlen pro Volumeneinheit Suspension mit dem Coulter counter bestimmt (vergl.Beispiel 6).

Beispiel 5

Herstellung einer 9% igen Nanosuspension mit RMKP 22/Lecithin - Durchmesser der Nanosuspension als Funktion der Zyklenzahl:

| Grundrezeptur | RMKP 22 | | 9,0 |
| --- | --- | --- | --- |
| | Glycerin 85% | | 2,5 |
| | Phopholipon | | 90 0,6 |
| | Aqua dest | ad | 100,0 |

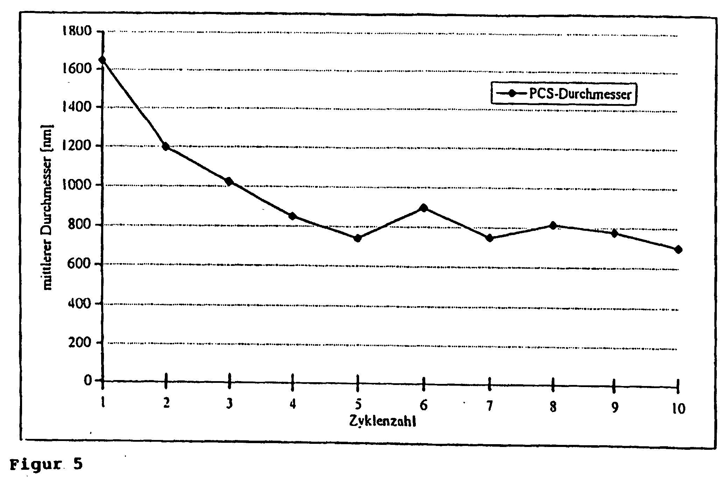

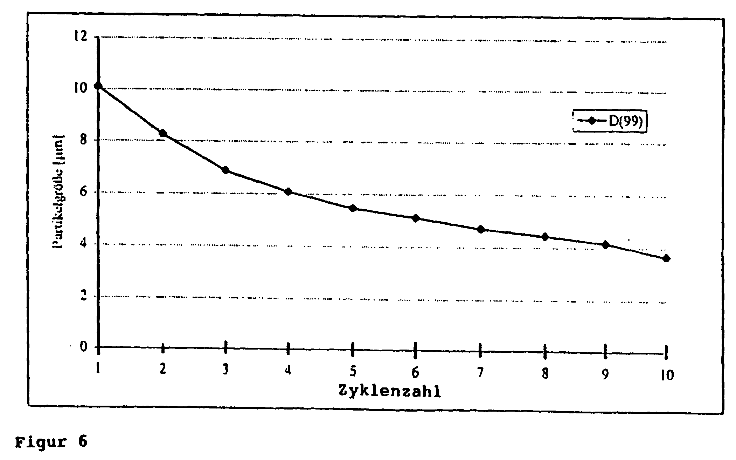

Herstellung der Suspension und anschließende Homogenisation wie in Beispiel 1. Homogenisationsparameter: 1500 bar, 1 bis 10 Zyklen. Die Darstellung des mittleren PCS - Durchmessers gegen die Zyklenzahl in Figur 5 ergibt einen fast minimalen Durchmesser der Nanosuspension nach 7 Zyklen, 780 nm. Um die Abnahme der Partikel auch im Größenbereich von 1 µm bis zu mehreren µm als Funktion der Zyklenzahl zu verfolgen, wurden die Proben im LD untersucht. Die Auswertung erfolgte über einen Auftrag des Durchmesser 99% gegen die Zyklenzahl (Figur 6) Hier wird ebenfalls nach ca. 7 Zyklen fast der minimale Durchmesser der Nanosuspension erzielt. Durchmesser 99% bedeutet, daß 99% der Partikel kleiner als dieser Wert sind (Volumenverteilung! , nicht Anzahlverteilung). Dieser Durchmesser ist ein sensitives Maß für die Reduzierung des Anteils an Mikrometerpartikeln. Nach 10 Zyklen ist auch hier die Grenzdispersität erreicht, 99 % der Teilchen sind < 3,87 µm, 100% sind < 5,29 µm.

Das Dispergier- und Mahlverhalten bei der Herstellung und die dabei erzielbaren Teilchgrößen sind bei Tween 80 und Phospholipon ähnlich.

Das Laser Diffraktometer liefert nur relative Verteilungen. Um die Injizierbarkeit von Nanosuspensionen zu beurteilen, wurden daher absolute Partikelzahlen pro Volumeneinheit Suspension mit dem Coulter counter bestimmt (vergl. Beispiel 6).

Beispiel 6

Herstellung einer 9%igen Nanosuspension mit RMKP 22/Tween 80 - Anteil an Partikeln im Mikrometerbereich und Beurteilung der i. v. Injizierbarkeit :

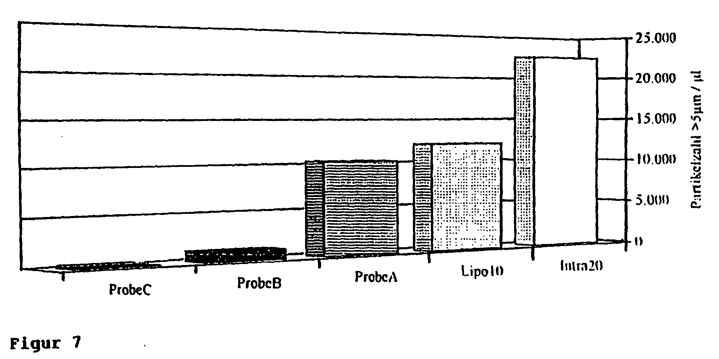

Das Laser Diffractometer liefert nur relative Verteilungen. Um die Injizierbarkeit von Nanosuspensionen zu beurteilen, wurden daher von den in Beispiel 4 hergestellten Nanosuspensionen die absolute Partikelzahlen pro Volumeneinheit Suspension mit dem Coulter counter Multisizer II bestimmt. Charakterisierunsparameter ist die Anzahl der Partikel > 5 μm pro μl Nanosuspension. In Abbildung 7 wurden vergleichend die Anzahl der Partikel > 5 μm pro μl Originalprobe der Nanosuspension A (9 % RMKP 22, 0,3% Tween 80, 16,7 % Mannitol, Aqua ad 100 Gew.%, Figur 7 Probe A) und der Fettemulsionen zur parenteralen Ernährung (Lipofundin 10% und Intralipid 20%, Figur 7 Lipo10 und Intra20) dargestellt. Weiterhin wurden Proben untersucht, deren Anzahl der Partikel > 5μm durch einen Zentrifugationsschritt gesenkt wurden. Die Nanosuspension B wurde 30 min bei 1559 g, die Nanosuspension C 30 min bei 3056 g zentrifugiert (Figur 7 : Probe B und Probe C).

Die Anzahl der Partikel in Mikrometerbereich liegt bei den zur i.v. Infusion zugelassenen Emulsionen (Infusionsvolumen > / = 500 ml p.d.) und der Nanosuspension A (Injektionsvolumen ca. 1-20 ml) bei 2,9 - 3 , 3 mg/ml. Gezielte Abscheidung von Partikeln > 5 μm durch Zentrifugation kann deren Anzahl bei den Nanosuspensionen B und C um ein Mehrfaches unter die Werte der Emulsionen auf 1,5 mg /ml senken (Figur 7: Probe B und Probe C, Probe A bei 1559 g bzw. 3056 g über 30 min zentrifugiert.).

Beispiel 7

Vergleich der Sättigungslöslichkeit von Mikropartikeln und Nanosuspensionen

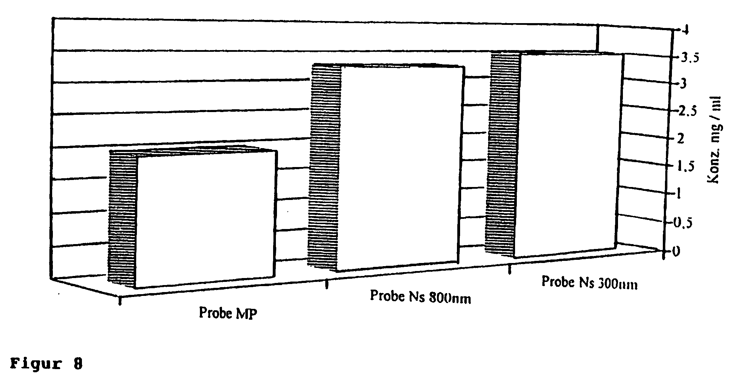

Die Bestimmung der Sättigungslöslichkeit Csm der Mikropartikel des luftstrahlgemahlenem Arzneistoffes RMKP 22 (Durchmesser 3,54 μm) erfolgte durch Schütteln sowohl in Wasser als auch in einer 0,3% iger Tween/16,7 %iger wäßriger Mannitlösung über 7 Tage. Nach 7 Tagen war ein Löslichkeitsplateau erreicht. Für beide Medien wurde eine identische Sättigungslöslichkeit gefunden, was Solubilisationseffekte für den Wirkstoff ausschließt. Die Bestimmung der Sättigungslöslichkeit Csn in zwei RMKP 22 - Nanosuspensionen (Durchmesser 800 nm und 300 nm) erfolgte im Dispersionsmedium (Tween 80/Mannitlösung) nach Abzentrifugieren der Feststoffphase. In der Figur 8 sind die Sättigungskonzentrationen von luftstrahlgemahlen RMKP 22 -Mikropartikeln (Probe MP, Durchmesser 2,40 μm) sowie von zwei RMKP 22 - Nanosuspensionen (Probe NS 800 nm, Probe NS 300 nm, mittlerer Durchmesser: 800 und 300 nm) gegenübergestellt. Die Sättigungslöslichkeit Csm der Mikropartikel liegt bei 1,97 mg/l und wird erst nach dreitägigem Schütteln erreicht. Dies bedeutet, daß sich der Arzneistoff sehr langsam auflöst. Es wurde kein signifikanter Unterschied zwischen der Sättigungslöslichkeit der beiden Pulver detektiert. Die Sättigungslöslichkeit der Nanosuspension wurde analog 7 Tage nach Herstellung bestimmt und ergab Werte von 3,29 mg/l und 3,52 mg/l. Eine Erhöhung der Sättigungslöslichkeit mit abnehmender Teilchengröße ist in der Gleichung von Ostwald-Freundlich beschrieben, wobei die gemessenen Werte jedoch nicht nur auf eine reine Vergrößerung der Oberfläche zurückgeführt werden.

Beispiel 8

Auflösungsverhalten von Nanosuspensionen im Vergleich zu Mikropartikeln:

| Grundrezeptur Nanosuspension: | | |
|---|---|---|
| RMKP 22 | | 9,0 |
| Tween 80 | | 0,3 |
| Mannit | | 16,7 |
| Aqua dest | ad | 100 |

Die Auflösung von Partikeln läßt sich mit einen Coulter counter bestimmen. Nach Einbringen von wenigen μl Partikelsuspension in das Meßvolumen von 100 ml kommt es bei löslicher Substanz im Laufe von drei aufeinanderfolgenden Wiederholungsmessungen zu einem Auflösen der Partikel, die Volumenkurve von Messung 1 bis 3 nimmt ab. Um Auflöseprozesse zu verhindern, mißt man bei derartigen Substanzen in einer mit Substanz gesättigter NaCl Lösung.

# EP 0 790 821 B1

Zur Herstellung einer mit Arzneistoff gesättigten Lösung wurde die 0,9% NaCl - Lösung mit luftstrahlgemahlenem Arzneistoff im Überschuß versetzt und durch Schütteln wurde die Sättigungslöslichkeit der Mikropartikel Csm erreicht. Die gesättigte Arzneistofflösung wurde gezielt nicht mit grobkristallinem sondern mit Arzneistoff-Mikropartikeln hergestellt, damit sich nach Ostwald - Freundlich auch die höhere Sättigungskonzentration Sättigungslöslichkeit über diesem feindispersen System einstellt.

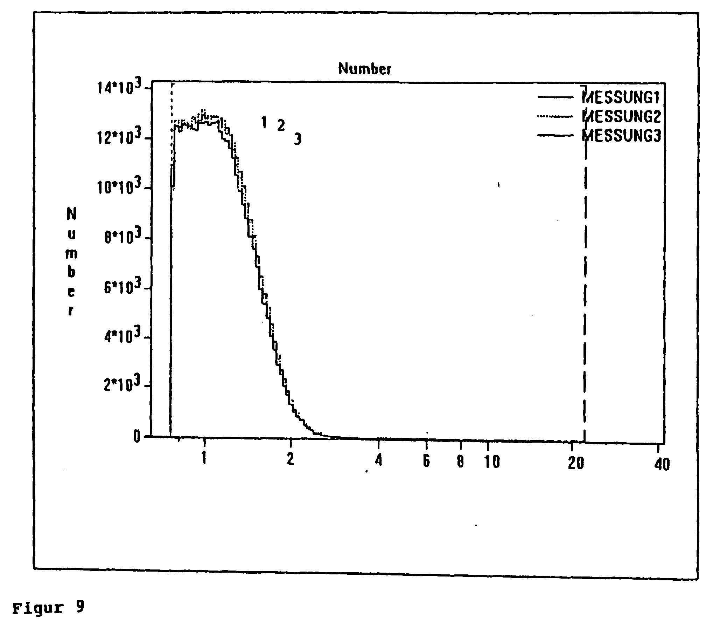

Das Einbringen von luftstrahlgemahlenen RMKP 22 Arzneistoffpartikeln, d. h. Partikeln mit einem Durchmesser 3,64 μm, in diese mit Arzneistoff gesättigte 0,9% NaCL Lösung führte demzufolge innerhalb der Meßzeit von ca. 10 Minuten (drei Wiederholungsmessungen a 150 s im Abstand von 100 s) zu keinerlei Auflösungserscheinungen, die drei nacheinander erhaltenen Meßkurven sind deckungsgleich (Figur 9). Das Gesamtvolumen der Partikel einer Probe beträgt während der ersten Messung 393.000 μm$^3$, während der zweiten Messung 391.400 μm$^3$ und dann 386.500 μm$^3$ (Figur 9). Das Gesamtvolumen der Partikel bleibt über den Zeitraum des Meßzykluses konstant.

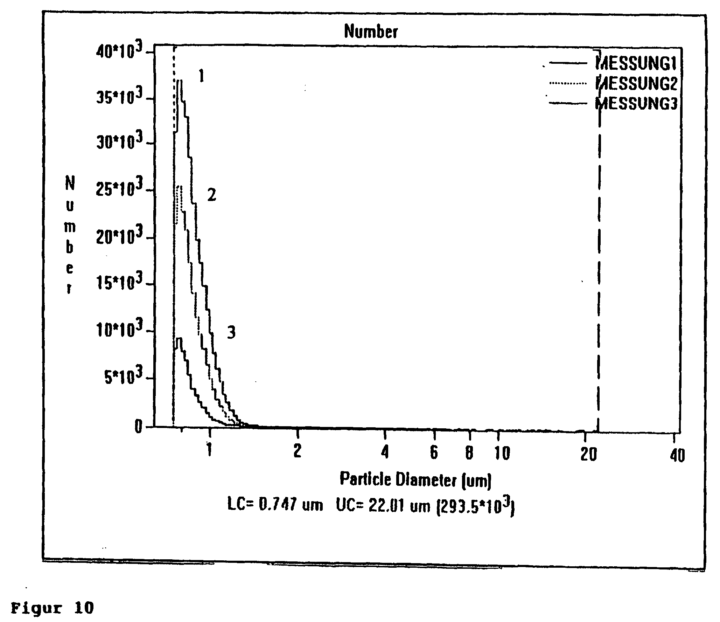

Das Vermessen der Nanosuspension, d. h. Partikeln in Nanometergröße, führte - trotz mit Arzneistoff gesättigter 0,9% NaCl-Lösung - zu einem Auflösen der Partikel innerhalb der Meßzeit von ca. 10 min, es lösten sich 65 % der Partikel auf. Die Coulter counter Volumenverteilung der Nanosuspension bei drei aufeinanderfolgenden Messungen (Start der Messung zu den Zeiten: T = 0s, T = 450 s, T = 1100 s, Dauer einer Messung: 150 s) ergibt ein Gesamtvolumen der Partikel während der ersten Messung von 121.000 μm$^3$, während der zweiten Messung von 83752 μm$^2$ und während der dritten Messung einen wert von 42038 μm$^3$ (Figur 10). Die abnehmende Fläche unter der Volumenverteilungskurve ist ein Maß für das Auflösen der Nanosuspension.

Eine über den Zeitraum eines Coulter counter Meßzykluses beobachtete Abnahme des Gesamtvolumens der Partikel einer Probe dokumentiert das Auflösungsverhalten der Nanopartikel im gewählten Meßmedium und zeigt das konstante Verhalten der mikronisierten Partikel im gleichen Meßmedium.

Beispiel 9

Lanagzeitstabilität von Nanosuspensionen

Grundrezepturen:

   A. 9% RMKP 22, 0,3% Tween 80, 16,7%, Mannit, Aqua dest ad 100%
   B. 9% RMKP 22, 1% Tween 80, 16,7%, Mannit, Aqua dest ad 100%
   C. 9% RMKP 22 , 0,6%, Phospholipon 90%, Aqua dest. ad 100%

Die Herstellung der Rezepturen erfolgte wie in Beispiel 1 beschrieben, Homogenisationsparameter: 1500 bar, 10 Zyklen. Analytik mit der PCS (Hauptdurchmesser) und mit dem Laser Diffractometer (Durchmesser 99% und 95%).

Die PCS Durchmesser und die dazugehörigen Polydispersitätsindices der gelagerten Nanosuspensionen betrugen:

| Charge A | 740 nm | 0,259 |
| Charge B | 719 nm | 0,282 |
| Charge C | 286 nm | 0,310. |

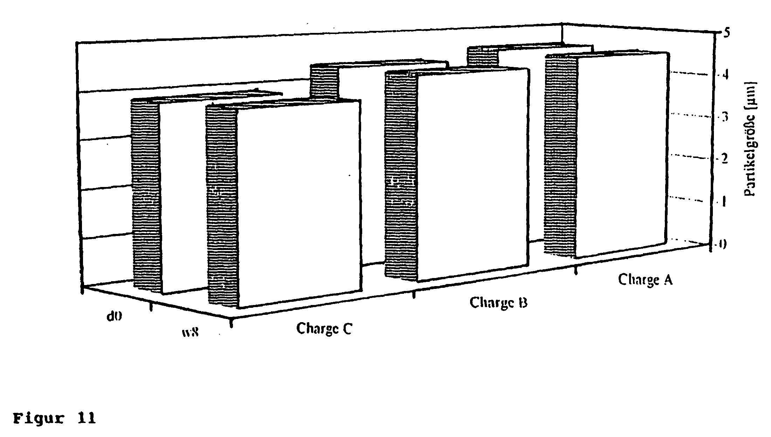

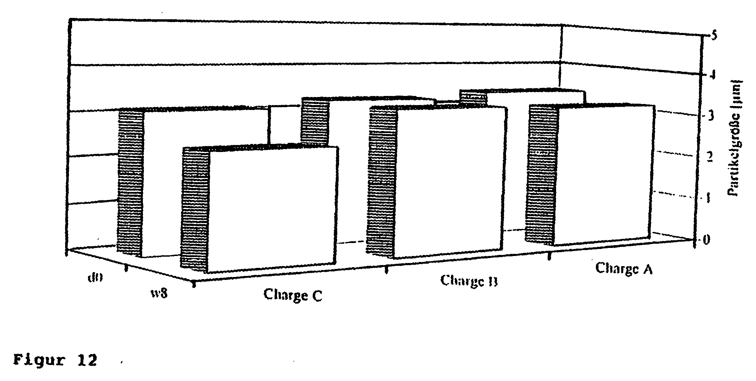

Durchmesser und Polydispersitätsindices zeigten keine signifikante Änderung der Partikelgrößenverteilung während der Lagerzeit. Auch die LD - Durchmesser 99% (Figur 11) und 95% (Figur 12) der Nanosusensionen A, B und C bleiben über eine Lagerzeit von 8 Wochen (w8) im Vergleich zu den Durchmessern am Herstellungstag (d0) konstant.

Beispiel 10

Stabilität von Nanosuspensionen bei der Sterilisation: Autoklavieren A121

Zusammensetzung der Stammsuspension A:

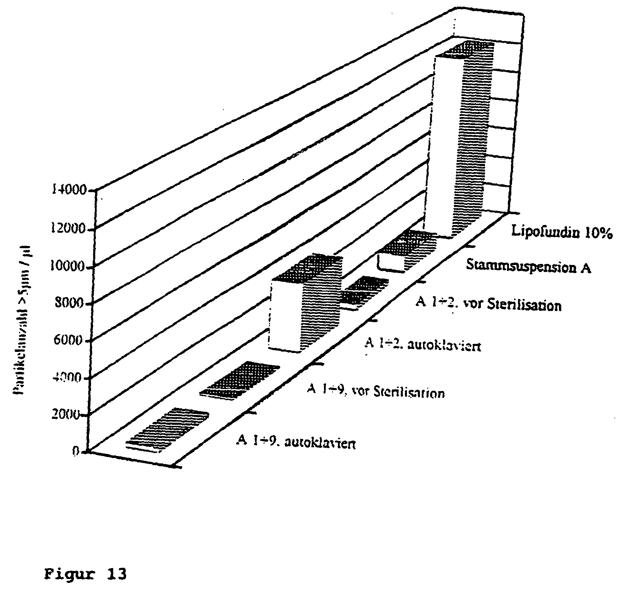

3% RMKP 22, 0,3% Tween 80, 16,7% Mannitol, Aqua dest ad 100 Gew%. Zur Sterilisation wurde die Stammsuspension A auf Arzneistoff-Anwendungskonzentrationen und damit auf die Tensidkonzentrationen von 1% (Figur 13: A 1 + 2) und auf 0,3% (Figur 13: A 1 + 9) mit Aqua dest. verdünnt. Die Sterilisation erfolgte mit gespanntem Dampf im Autoklaven nach dem Deutschen Arzneibuch, 10. Ausgabe (15 Minuten, 121° C bei 2 bar). Die Partikelanalytik erfolgte mit dem Coulter counter und mit der PCS.

Die Figur 13 zeigt die Coulter Counter Ergebnisse von der Stämmsuspension A (Figur 13: Stammsuspension A), von den Nanosuspensionen A 1÷2 und A 1÷9 vor der Sterilisation (Figur 13: A 1 ÷ 2 / 9 , vor Sterilisation) und nach der Sterilisation (Figur 13: A 1 ÷ 2 / +9, autoklaviert). Als Vergleich sind die Anzahl der Partikel > 5µm pro µl in Lipofundin 10% (Figur 13: Lipofundin 10%) herangezogen worden.

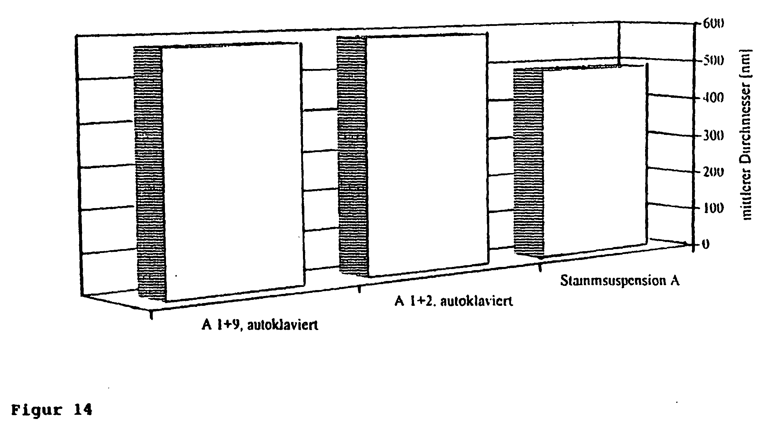

PCS - Daten ergeben den Partikelhauptdurchmesser der Stammsuspension A sowie die Hauptdurchmesser der Nanosuspensionen A 1÷2 und A 1÷9 nach dem Autoklavieren (Figur 14: A 1 ÷ 2 / ÷9, autoklaviert).

Die Anzahl der Partikel größer 5 µm stieg infolge der Temperaturbelastung der Nanosuspensionen und daduch entstehender Aggregatbildung an. In der mit 2 Teilen Wasser verdünnten Nanosuspension A 1÷2 erhöhte sich die Anzahl an Partikeln > 5 µm über den Wert der höher konzentrierten, nicht sterilisierten Stammsuspension A, blieb jedoch noch deutlich unter den Werten der Fettemulsionen. Verdünnung mit 9 Teilen Wasser senkte die Wahrscheinlichkeit der Kollision zweier Partikel durch die Verringerung der Partikelkonzentration so stark, daß ein signifikanter Anstieg in der Anzahl an Partikeln vor und nach Sterilisation nicht mehr detektierbar war. Die Durchmesser erhöhten sich beim Autoklavieren um 98 nm bzw 91 nm (A 1÷2 / A 1÷9), was die i.v. Injizierbarkeit nicht beeinträchtigt (Figur 14).

Beispiel 11

Stabilität von Nanosuspensionen bei der Sterilisation: Gamma-Sterilisation

Zusammensetzung der Nanosuspensionen A und B:

Nanosuspension A: 2% RMKP, 0,3% Tween 80, 16,7% Mannitol, Aqua dest ad 100 Gew%.

Nanosuspension B: 3% RMKP, 0,3% Tween 80, 16,7% Mannitol, Aqua dest ad 100 Gew%.

Die Nanosuspensionen A und B wurden mit einer Kobalt-60 Quelle und einer Dosis von 2,5 Mrad (25 kGray) sterilisiert. Die Analytik erfolgte mit dem Coulter counter Multisizer II und der PCS.

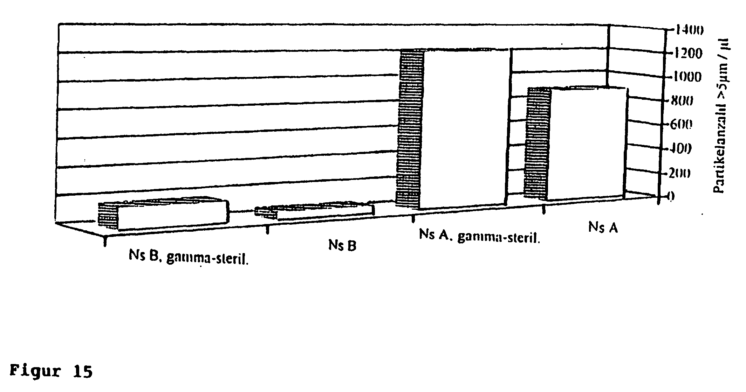

Die Anzahl an Partikeln > 5 µm pro µl der Nanosuspensionen A und B vor der Sterilisation und nach der Sterilisation (Figur 15: : Ns A, Ns B / Ns A, gamma-steril., Ns B, gamma-steril.) werden mit dem Coulter counter erfaßt (Figur 15). Als Vergleich dienen die Partikelanzahlen in Lipofundin 10% und Intralipid 20%:
12.176 und 22.525 Partikel > 5 µm pro µl Emulsion.

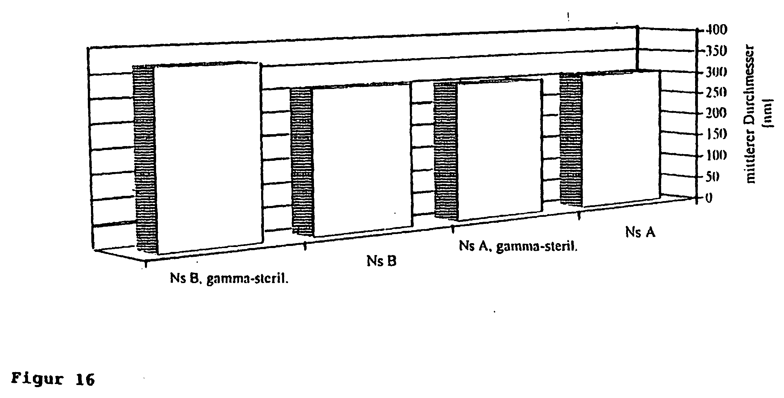

Die PCS Partikeldurchmesser der Nanosuspensionen A und B vor (NS A / NS B) und nach der Sterilisation (NS A, gamma-steril., NS B, gamma-steril,) sind in der Figur 16 aufgeführt.

Es kam zu einer moderaten Erhöhung der Partikel > 5 µm bei der Sterilisation, bei der Nanosuspension A von 890 auf 1222, bei der Nanosuspension B von 60 auf 165, wobei die Anzählen deutlich auch nach der Sterilisation noch unterhalb der Werte in den Fettemulsionen bleiben. Der PCS Durchmesser steigt bei der Nanosuspension A nicht an (303 nm vor, 299 nach Sterilisation), bei Nanosuspension B geringfügig (von 306 auf 367 nm) . Die Partikeldurchmesser bei parenteralen Fettemulsionen bewegen sich im Bereich von ca. 200 bis 400 nm.

Beispiel 12

Stabilität von Nanosuspensionen bei der Sterilisation als Funktion der Tensidkonzentration

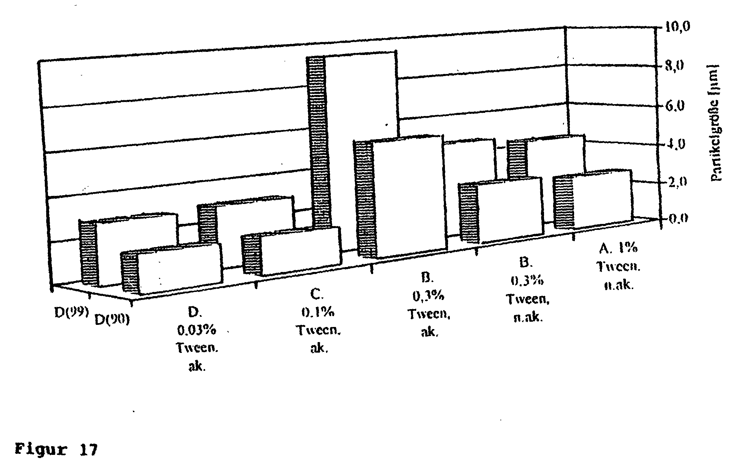

Nanosuspensionen aus RMKP, stabilisiert mit unterschiedlichen Tween 80 Konzentrationen, wurden mit A121 sterilisert und mit dem Laser Diffraktometer bezüglich Teilchenwachstum analysiert (Figur 17). Zusammensetzung der Nanosuspensionen:

A. 1,0% Tween, 9% RMKP, Mannit 16,7%
B. 0,30% Tween, 9% RMKP, Mannit 16,7%
C. 0,10% Tween, 0,9% RMKP, Mannit 16,7%
D. 0,03% Tween, 0,9% RMKP, Mannit 16,7%

Die Rezepturen enthielten jeweils Aqua dest. ad 100 Gew%, die Nanosuspensionen C und D wurden aus der Stammsuspension B durch Verdünnen auf Gebrauchskonzentration hergestellt. Bei der Nanosuspension C wurde nach dem Verdünnen Tween 80 zugesetz, um auf 0,10% einzustellen.

Als Charakterisierungsgrößen für das Teilchenwachstum dienen die LD - Durchmesser 99% und 90% von Nanosuspensionen mit unterschiedlicher Tween 80 Konzentration vor und nach dem Autoklavieren (Figur 17: n. ak / ak.). Daten von der Nanosuspension B (Figur 17: B, 0,3%Tween 80 n.ak) sind die Ausgangswerte der Suspensionen C und D vor dem Autoklavieren (Figur 17).

Die Nanosuspension mit 1% Tween 80 zeigte nach dem Autoklavieren bereits makroskopische sichtbare Aggregate und wurde daher nicht mehr mittels des Laser Diffractometers untersucht. Überraschenderweise zeigten die Nanosuspensionen mit abnehmender Tensidkonzentration eine höhere Stabilität.

Beispiel 13

Tensidfreie Nanosuspensionen mit Carbamazepin

| Grundrezeptur | Carbamazepin | | 1,0 |
|---|---|---|---|
| | Natriumcarboxymethylcellulose | | 0,1 |
| | Aqua dest | ad | 100,0 |

Natriumcarboxymethylcellulose wurde in Wasser gelöst und der gepulverte Wirkstoff damit in einer Reibschale angerieben. Der Ansatz wurde 2min im Ultraturrax dispergiert. Diese grobe Vordispersion wurde dann mit 1500 bar und 5 Zyklen homogenisiert.

| Kenndaten der Nanosuspension | 436 nm Durchmesser |
|---|---|
| | 0,263 Polydispersitätsindex |

Vergleichsbeispiel 14

Tetracain Nanosuspension hergestellt mit Scher- und Pralldispergierung (jet stream)

| Grundrezeptur | Tetracain Base | | 1,0 |
|---|---|---|---|
| | Lecithin S 75 | | 0,3 |
| | Pluronic F68 | | 2,2 |
| | Glycerol 85% | | 2,2 |
| | Aqua dest. | ad | 100,0 |

Tetracain Base wird mit der Pluronic Lösung angerieben und anschließend mit einem Druck von 600 bar und in 5 Zyklen durch den Microfluidizer Modell 110 - Y (Microfluidics Inc.) gegeben. Es wurde mit diesem Dispergierprinzip ebenfalls eine Nanosuspension erhalten.

| Kenndaten der Nanosuspension | 379 nm Durchmesser |
|---|---|
| | 0,529 Polydispersitätsindex |

Beispiel 15

Tetracain Nanosuspension < 100 nm hergestellt mit Kavitation

| Grundrezeptur | Tetracain Base | | 1,0 |
|---|---|---|---|
| | Pluronic F68 | | 2,2 |
| | Lecithin S75 | | 0,3 |
| | Glycerol 85% | | 2,2 |
| | Aqua dest | ad | 100,0 |

Die Herstellung erfolgte wie in Beispiel 1 beschrieben, Homogenisationsparameter: 1500 bar und 10 Zyklen.

Analytik wurde mit PCS vorgenommen.

| Kenndaten der Nanosuspension | 91 nm Durchmesser |
|---|---|
| | 0.489 Polydispersitätsindex |

Durch die spezielle Zusammensetzung der Rezeptur (niedrige Konzentration der dispersen Phase) wurden Nanosuspensionen mit einer Partikelgröße unter 100 nm erhalten, die ein potentielles System für das Targeting z.B. zu Ehdothelzellen der Blutkapillaren sind (Partikelaufnahme erfolgt hier durch Pinocytose, die auf Partikel < 150 nm beschränkt ist).

Beispiel 16

Prednisolon Nanosuspension < 100 nm hergestellt mit Kavitation

| Grundrezeptur | Prednisolon | | 1,0 |
|---|---|---|---|
| | Pluronic F68 | | 2,2 |
| | Lecithin S75 | | 0,3 |
| | Glycerol 85% | | 2,2 |
| | Aqua. dest | ad | 100,0 |

Die Herstellung erfolgte wie in Beispiel 1 beschrieben, Homogenisationsparameter: 1500 bar und 10 Zyklen. Analytik wurde mit PCS und Laser Diffraktometer (LD) vorgenommen.

| Kenndaten der Nanosuspension | 897 nm Durchmesser |
|---|---|
| | 0,040 Polydispersitätsindex |
| | 3,80 Durchmesser 95% (LD) |
| | 4,74 µm Durchmesser 99%(LD) |

**Patentansprüche**

1. Verfahren zur Herstellung eines Arzneistoffträgers, der Teilchen aus in Wasser unlöslichem oder nur wenig löslichem reinem Wirkstoff oder aus einem Gemisch von 2 oder mehr solcher Wirkstoffe umfasst, die bei Raumtemperatur fest sind, **dadurch gekennzeichnet, dass** man eine Suspension des Wirkstoffes oder Wirkstoffgemisches in Wasser oder wässrigem Medium einer Hochdruckhomogenisation in einem Kolben-Spalt-Homogenisator unterwirft, um Teilchen zu bilden, die einen mit Photonenkorrelationsspektroskopie bestimmten mittleren Durchmesser von 10 nm bis 1000 nm aufweisen, wobei der Anteil an Partikeln grösser als 5 µm in der Gesamtpopulation, bezogen auf die mit Coulter counter ermittelte Anzahlverteilung, kleiner als 0.1% ist.

2. Verfahren nach Anspruch 1, worin der Arzneistoff bei. Einbringung in Wasser oder wässrige Flüssigkeiten eine im Vergleich zu Pulvern des Arzneistoffes erhöhte Sättigungslöslichkeit und eine erhöhte Lösungsgeschwindigkeit aufweist.

3. Verfahren nach Anspruch 1, worin die Teilchen der Hauptpopulation einen mittleren Durchmesser zwischen 40 und 1000 nm, insbesondere von 100 bis 800 nm, und bei geeigneter Verfahrensparameter- und Hilfsstoffauswahl zwischen 40 und 100 nm aufweisen.

4. Verfahren nach Anspruch 1, worin die Herstellung unter Ausschluss von Tensiden erfolgt.

5. Verfahren nach Anspruch 1, worin die Herstellung unter Verwendung von synthetischen, halbsynthetischen und/ oder natürlichen Tensiden, wie Lecithin oder natürlichem, aufgereinigtem Lecithin, in Konzentrationen von 0,001 bis 30% erfolgt.

6. Verfahren nach Anspruch 5, worin die Tensidkonzentration kleiner als 1,0%, insbesondere kleiner als 0,5% ist.

7. Verfahren nach Anspruch 1, 5 oder 6, worin die Herstellung unter Verwendung von Tensiden in Mischung mit einem oder mehreren anderen Stabilisatoren erfolgt.

8. Verfahren nach Anspruch 1, worin die Herstellung unter Ausschluss der Verwendung von organischen Lösungsmitteln erfolgt.

9. Verfahren nach Anspruch 1, worin die Herstellung ohne Verwendung von Ultraschallstäben, Kugel- oder Perlmühlen erfolgt.

10. Verfahren nach einem der Ansprüche 1 bis 9, worin der Anteil der inneren oder Arzneistoffphase bezogen auf die Grundrezeptur 0,1 bis 30 Gew.%, insbesondere 1 bis 20 Gew.%, beträgt.

11. Verfahren nach einem der Ansprüche 1 bis 10, worin der Arzneistoffträger aus einem Wirkstoff oder Wirkstoffen besteht, die in Wasser oder wässrigen Lösungen gering löslich oder unlöslich sind.

12. Verfahren nach einem der Ansprüche 1 bis 10, worin der Arzneistoffträger aus einem Wirkstoff oder Wirkstoffen besteht, die in organischen Lösungsmitteln gering löslich oder unlöslich sind.

13. Verfahren nach einem der Ansprüche 1 bis 10, worin der Arzneistoffträger aus einem Wirkstoff oder Wirkstoffen besteht, die in Wasser oder wässrigen Lösungen und in organischen Lösungsmitteln gering löslich oder unlöslich sind.

14. Verfahren nach einem der Ansprüche 1 bis 10, worin der Arzneistoffträger aus einem Wirkstoff oder Wirkstoffen besteht, die in organischen Lösungsmitteln eine mittlere Löslichkeit besitzen.

15. Verfahren nach einem der Ansprüche 1 bis 14, worin der Arzneistoffträger ausserdem eine oder mehrere dispersionsstabilisierende Substanzen umfasst.

16. Verfahren nach Anspruch 15, worin die dispersionsstabilisierenden Substanzen in einer Menge von 0,001 bis 20 Gew.%, insbesondere 0,01 bis 5 Gew.%, bezogen auf die Grundrezeptur, vorliegen.

17. Verfahren nach Anspruch 15 oder 16, worin die stabilisierenden Substanzen Verbindungen aus der Reihe der Poloxamere, Poloxamine, ethoxylierten Mono- und Diglyceride, ethoxylierten Lipide und Lipoide, ethoxylierten Fettalkohole und Alkylphenole, ethoxylierten Fettsäureester, Polyglycerinether und -ester, Lecithine, Ester und Ether von Zuckern oder Zuckeralkoholen mit Fettsäuren oder Fettalkoholen, Phospholipide und Sphingolipide, Sterine, deren Ester oder Ether sowie deren Mischungen dieser Verbindungen umfassen.

18. Verfahren nach einem der Ansprüche 15 bis 17, worin die stabilisierende Substanz Eilecithin, Sojalecithin oder hydriertes Lecithin, deren Mischungen oder Mischungen aus einem oder beiden Lecithinen mit einer oder mehreren Phospholipidkomponenten, Cholesterin, Cholesterinpalmitat; Stigmasterin oder andere Sterine umfasst.

19. Verfahren nach einem der Ansprüche 1 bis 18, worin der Arzneistoffträger ausserdem Ladungsstabilisatoren umfasst.

20. Verfahren nach Anspruch 19, worin der Arzneistoffträger Ladungsstabilisatoren in einer Menge von 0,01 bis 20 Gew.%, insbesondere 0,01 bis 2 Gew.%, bezogen auf die Grundrezeptur, umfasst.

21. Verfahren nach Anspruch 19 oder 20, worin die Ladungsstabilisatoren Dicetylphosphat, Phosphatidylglycerol, gesättigte oder ungesättigte Fettsäuren, Natriumcholat, Peptisatoren oder Aminosäuren umfassen.

22. Verfahren nach einem der Ansprüche 1 bis 14, worin der Arzneistoffträger einen oder mehrere viskositätserhöhende Stoffe umfasst.

23. Verfahren nach Anspruch 22, worin die viskositätserhöhenden Stoffe in einer Menge von 0,1 bis 20 Gew.%, insbesondere 0,5 bis 5 Gew.%, bezogen auf die Grundrezeptur, vorliegen.

24. Verfahren nach Anspruch 22 oder 23, worin die viskositätserhöhenden Stoffe Celluloseether und -ester, Polyvinylderivate, Polyvinylalkohol, Alginate, Xanthane, Pektine, Polyacrylate, Poloxamere und Poloxamine umfassen.

25. Verfahren nach einem der Ansprüche 22 bis 24, worin der Arzneistoffträger ausserdem Zucker oder Zuckeralkohole, insbesondere Glucose, Mannose, Trehalose, Mannit und Sorbit umfasst.

26. Verfahren nach einem der Ansprüche 22 bis 25, worin der Arzneistoffträger ausserdem Ladungsträger umfasst.

27. Verfahren nach einem der Ansprüche 1 bis 26, worin die Teilchen in destilliertem Wasser oder einem wässrigen Medium oder in einem wässrigen Medium mit Zusätzen aus Elektrolyten, Mono- und Disacchariden, Polyolen oder deren Mischungen dispergiert sind.

28. Verfahren nach Anspruch 27, worin die Zusätze Natriumchlorid, Mannose, Glucose, Fructose, Xylose, Mannit, Sorbit, Xylit und Glycerin umfassen.

29. Verfahren nach einem der Ansprüche 1 bis 28, worin die Teilchen lyophilisiert oder sprühgetrocknet werden.

30. Verfahren nach einem der Ansprüche 1. bis 29, worin der Arzneistoffträger aus einem oder mehreren Wirkstoffen besteht.

31. Verfahren nach Anspruch 30, worin bei mehreren Wirkstoffen ein Wirkstoff oder mehrere Wirkstoffe in einem anderen oder mehreren anderen gelöst sind (sog. feste Lösung) oder dispergiert sind (sog. feste Dispersion), an deren Oberflächen adsorbiert sind oder als Lösung in dem Teilchen dispergiert sind.

32. Verfahren nach einem der Ansprüche 1 bis 26, worin die Teilchen in einem nichtwässrigen Medium dispergiert sind.

33. Verfahren nach Anspruch 32, worin die Teilchen in einem flüssigen, halbfesten oder festen Medium dispergiert sind.

34. Verfahren nach Anspruch 33, worin die Teilchen in einem flüssigen öligen Medium wie Ricinusöl, Erdnussöl, Olivenöl, Neutralöl (Miglyol 812), Sesamöl, Maisöl, Baumwollsamenöl, Mandelöl, Mittelkettige Triglyceride oder anderen Ölen dispergiert sind.

35. Verfahren nach Anspruch 33, worin das Medium aus Lipiden oder Lipoiden oder deren Mischungen besteht.

36. Verfahren nach Anspruch 35, worin das Medium aus Mono-, Di-, Triglyceriden (z.B. Witepsole, Softisane), Wachsen, Fettalkoholen und Fettalkoholestern, Bienenwachs, ölsäureoleylester, Isopropylmyristat, Wollwachs oder deren Mischungen besteht.

37. Verfahren nach Anspruch 33, worin das Medium aus längerkettigen organischen Molekülen oder Polymeren, aus flüssigen, halbfesten oder festen Polyethylenglykolen, Poloxameren, Poloxaminen oder deren Mischungen besteht.

38. Verfahren nach einem der Ansprüche 1 bis 37, worin der Arneistoff oder die Arzneistoffmischung zu einem Pulver gemahlen, in einem Dispersionsmittel, insbesondere Wasser oder wässrigem Medium, dispergiert und dann der Hochdruckhomogenisation unterworfen wird.

**Claims**

1. Method for production of a drug carrier, which comprises particles of pure active ingredient which is water insoluble or only slightly soluble or of a mixture of two or more such active ingredients, which are solid at room temperature, **characterised in that** a suspension of the active ingredient or active ingredient mixture in water or aqueous medium is subjected to a high pressure homogenisation in a piston-gap homogeniser, in order to form particles, which have an average diameter of 10 nm to 1000 nm determined by photon correlation spectroscopy, wherein the proportion of particles larger than 5μm in the total population is less than 0.1%, based on the number distribution determined by Coulter counter.

2. Method according to claim 1, wherein the drug carrier when introduced into water or aqueous liquids, has increased saturation solubility and an increased rate of dissolution, in comparison to powders of the drug carrier.

3. Method according to claim 1, wherein the particles of the main population have an average diameter of between 40 and 1000 nm, in particular from 100 to 800 nm, and with suitable choice of method parameters and auxiliary material of between 40 and 100 nm.

4. Method according to claim 1, wherein the production occurs with the exclusion of tensides.

5. Method according to claim 1, wherein the production occurs with the use of synthetic, semi-synthetic and/or naturally occurring tensides, such as lecithin or natural, purified lecithin, in concentrations from 0.001 to 30%.

6. Method according to claim 5, wherein the tenside concentration is less than 1.0%, in particular less than 0.5%.

7. Method according to claim 1,5 or 6, wherein the production occurs with the use of tensides in mixture with one or several other stabilisers.

8. Method according to claim 1, wherein the production occurs with the exclusion of the use of organic solvents.

9. Method according to claim 1, wherein the production occurs without use of ultrasonic probes, ball mills or pearl mills.

10. Method according to any one of claims 1 to 9, wherein the proportion of the inner or drug phase, based on the basic recipe, is 0.1 to 30 wt.%, in particular 1 to 20 wt.%.

11. Method according to any one of claims 1 to 10, wherein the drug carrier consists of an active ingredient or active ingredients, which are slightly soluble or insoluble in water or aqueous solutions.

12. Method according to any one of claims 1 to 10, wherein the drug carrier consists of an active ingredient or active ingredients, which are slightly soluble or insoluble in organic solvents.

13. Method according to any one of claims 1 to 10, wherein the drug carrier consists of an active ingredient or active ingredients, which are slightly soluble or insoluble in water or aqueous solutions and in organic solvents.

14. Method according to any one of claims 1 to 10, wherein the drug carrier consists of an active ingredient or active ingredients, which possess a moderate solubility in organic solvents.

15. Method according to any one of claims 1 to 14, wherein the drug carrier comprises in addition one or several dispersion-stabilising substances.

16. Method according to claim 15, wherein the dispersion-stabilising substances are present in an amount from 0.001 to 20 wt.%, in particular 0.01 to 5 wt.%, based on the basic recipe.

17. Method according to claim 15 or 16, wherein the stabilising substances comprise compounds from the series of poloxamers, poloxamines, ethoxylated mono- and diglycerides, ethoxylated lipids and lipoids, ethoxylated fatty alcohols and alkyl phenols, ethoxylated fatty acid esters, polyglycerol ethers and esters, lecithins, esters and ethers of sugars or sugar alcohols with fatty acids or fatty alcohols, phospholipids and sphingolipids, sterols, esters or ethers thereof and mixtures thereof of these compounds.

18. Method according to any one of claims 15 to 17, wherein the stabilising substance comprises egg lecithin, soy lecithin or hydrogenated lecithin, mixtures thereof or mixtures of one or both lecithins with one or several phospholipid components, cholesterol, cholesterol palmitate or stigmasterol or other sterols.

19. Method according to any one of claims 1 to 18, wherein the drug carrier comprises in addition charge stabilisers.

20. Method according to claim 19, wherein the drug carrier comprises charge stabilisers in an amount from 0.01 to 20 wt.%, in particular 0.01 to 2 wt.%, based on the basic recipe.

21. Method according to claim 19 or 20, wherein the charge stabilisers comprise dicetyl phosphate, phosphatidylglycerol, saturated or unsaturated fatty acids, sodium cholate, peptisers or amino acids.

22. Method according to any one of claims 1 to 14, wherein the drug carrier comprises one or more viscosity-increasing substances.

23. Method according to claim 22, wherein the viscosity-increasing substances are present in an amount from 0.1 to 20 wt.%, in particular 0.5 to 5 wt.%, based on the basic recipe.

24. Method according to claim 22 or 23, wherein the viscosity-increasing substances comprise cellulose ethers and esters, polyvinyl derivatives, polyvinyl alcohol, alginates, xanthans, pectins, polyacrylates, poloxamers and poloxamines.

25. Method according to any one of claims 22 to 24, wherein the drug carrier comprises in addition sugar or sugar alcohols, in particular glucose, mannose, trehalose, mannitol and sorbitol.

26. Method according to any one of claims 22 to 25, wherein the drug carrier comprises in addition charge carriers.

27. Method according to any one of claims 1 to 26, wherein the particles are dispersed in distilled water or an aqueous medium or in an aqueous medium with additions of electrolytes, mono- and disaccharides, polyols or mixtures thereof.

28. Method according to claim 27, wherein the additions comprise sodium chloride, mannose, glucose, fructose, xylose, mannitol, sorbitol, xylitol and glycerol.

29. Method according to any one of claims 1 to 28, wherein the particles are lyophilised or spray dried.

30. Method according to any one of claims 1 to 29, wherein the drug carrier consists of one or several active ingredients.

31. Method according to claim 30, wherein in the case of several active ingredients, one active ingredient or several active ingredients are dissolved (so called solid solution) or dispersed (so called solid dispersion) in another or several others, adsorbed onto the surface thereof or dispersed as a solution in the particle.

32. Method according to any one of claims 1 to 26, wherein the particles are dispersed in a non-aqueous medium.

33. Method according to claim 32, wherein the particles are dispersed in a liquid, semi-solid or solid medium.

34. Method according to claim 33, wherein the particles are dispersed in a liquid oily medium such as ricinus oil, peanut oil, olive oil, neutral oil (Miglyol 812), sesame oil, maize oil, cotton seed oil, almond oil, medium chain triglycerides or other oils.

35. Method according to claim 33, wherein the medium consists of lipids or lipoids or mixtures thereof.

36. Method according to claim 35, wherein the medium consists of mono-, di-, triglycerides (e.g. witepsoles, softisans), waxes, fatty alcohols and fatty alcohol esters, beeswax, oil acid oleyl ester, isopropyl myristate, wool wax or mixtures thereof.

37. Method according to claim 33, wherein the medium consists of longer chain organic molecules or polymers, of liquid, semi-solid or solid polyethylene glycols, poloxamers, polxamines or mixtures thereof.

38. Method according to any one of claims 1 to 37, wherein the drug or drug mixture is ground to a powder, dispersed in a dispersion agent, in particular water or aqueous medium, and is then subjected to the high pressure homogenisation.

**Revendications**

1. Procédé de fabrication d'un excipient de médicament, qui comprend des particules d'un agent actif pur non soluble ou seulement peu soluble dans l'eau ou un mélange de 2 ou de plusieurs agents actifs de ce genre, qui sont solides à la température ambiante, **caractérisé en ce que** l'on soumet une suspension de l'agent actif ou du mélange d'agents actifs, dans l'eau ou dans un milieu aqueux, à une homogénéisation haute pression dans un homogénéisateur piston-fente, pour former des particules qui présentent un diamètre moyen, déterminé par spectroscopie de corrélation de photons, de 10 nm à 1000 nm, la proportion en particules supérieures à 5 µm dans la population totale, par rapport à la distribution numérique déterminée à l'aide d'un compteur Coulter, étant inférieure à 0,1%.

2. Procédé selon la revendication 1, dans lequel le médicament présente, lors de l'introduction dans l'eau ou dans des liquides aqueux, une solubilité de saturation plus élevée et une vitesse de dissolution plus élevée par rapport aux poudres du médicament.

3. Procédé selon la revendication 1, dans lequel les particules de la population principale présentent un diamètre moyen compris entre 40 et 1000 nm, en particulier de 100 à 800 nm, et, dans le cas d'un choix approprié de paramètres de procédé et d'adjuvants, entre 40 et 100 nm.

4. Procédé selon la revendication 1, dans lequel la fabrication se fait en l'absence d'agents tensioactifs.

5. Procédé selon la revendication 1, dans lequel la fabrication se fait par utilisation d'agents tensioactifs synthétiques, semi-synthétiques et/ou naturels, comme la lécithine ou la lécithine purifiée naturelle, dans des concentrations de 0,001 à 30%.

6. Procédé selon la revendication 5, dans lequel la concentration en agents actifs est inférieure à 1,0%, en particulier inférieure à 0,5%.

7. Procédé selon la revendication 1, 5 ou 6, dans lequel la fabrication se fait par utilisation d'agents tensioactifs en mélange à un ou plusieurs agents de stabilisation.

8. Procédé selon la revendication 1, dans lequel la fabrication se fait en l'absence de solvants organiques.

9. Procédé selon la revendication 1, dans lequel la fabrication se fait sans utilisation de tiges ultrasoniques, de broyeurs à boulets ou moulins à perles.

10. Procédé selon l'une quelconque des revendications 1 à 9, dans lequel la proportion de la phase interne ou de la phase de médicament, par rapport à la formulation de base, est de 0,1 à 30% en poids, en particulier de 1 à 20% en poids.

11. Procédé selon l'une quelconque des revendications 1 à 10, dans lequel l'excipient de médicament se compose d'un agent actif ou d'agents actifs, qui sont faiblement solubles ou insolubles dans l'eau ou les solutions aqueuses.

12. Procédé selon l'une quelconque des revendications 1 à 10, dans lequel l'excipient de médicament se compose d'un agent actif ou d'agents actifs, qui sont faiblement solubles ou insolubles dans les solvants organiques.

13. Procédé selon l'une quelconque des revendications 1 à 10, dans lequel l'excipient de médicament se compose d'un agent actif ou d'agents actifs, qui sont faiblement solubles ou insolubles dans l'eau ou dans les solutions aqueuses et dans les solvants organiques.

14. Procédé selon l'une quelconque des revendications 1 à 10, dans lequel l'excipient de médicament se compose d'un agent actif ou d'agents actifs, qui possèdent une solubilité moyenne dans les solvants organiques.

15. Procédé selon l'une quelconque des revendications 1 à 14, dans lequel l'excipient de médicament comprend en outre une ou plusieurs substances de stabilisation des dispersions.

16. Procédé selon la revendication 15, dans lequel les substances de stabilisation des dispersions sont présentes dans une quantité de 0,001 à 20% en poids, en particulier de 0,01 à 5% en poids, par rapport à la formulation de base.

17. Procédé selon la revendication 15 ou 16, dans lequel les substances de stabilisation comprennent des composés choisis parmi la série des poloxamères, des poloxamines, des monoglycérides et des diglycérides éthoxylés, des lipides et des lipoïdes éthoxylés, des alcools gras et des alkylphénols éthoxylés, des esters d'acides gras éthoxylés, des éthers et des esters de la polyglycérine, de la lécithine, des esters et des éthers de sucres ou d'alcools de sucre avec des acides gras ou avec des alcools gras, des phospholipides et des sphingolipides, des stérines, de leurs éthers ou leurs esters ainsi que des mélanges de ces composés.

**18.** Procédé selon l'une quelconque des revendications 15 à 17, dans lequel la substance de stabilisation comprend la lécithine de l'oeuf, la lécithine du soja ou la lécithine hydrogénée, leurs mélanges ou des mélanges d'une ou des deux lécithines avec un ou plusieurs composants de phospholipides, la cholestérine, le palmitate de cholestérine, la stigmastérine ou d'autres stérines.

**19.** Procédé selon l'une quelconque des revendications 1 à 18, dans lequel l'excipient de médicament comprend en outre des agents de stabilisation des charges.

**20.** Procédé selon la revendication 19, dans lequel l'excipient de médicament comprend des agents de stabilisation des charges dans une quantité de 0,01 à 20% en poids, en particulier de 0,01 à 2% en poids, par rapport à la formulation de base.

**21.** Procédé selon la revendication 19 ou 20, dans lequel les agents de stabilisation des charges comprennent le phosphate de dicétyle, le phosphatidylglycérol, des acides gras saturés ou insaturés, le cholate de sodium, des agents dispersants ou des aminoacides.

**22.** Procédé selon l'une quelconque des revendications 1 à 14, dans lequel l'excipient de médicament comprend en outre une ou plusieurs substances d'augmentation de la viscosité.

**23.** Procédé selon la revendication 22, dans lequel les substances d'augmentation de la viscosité sont présentes dans une quantité de 0,1 à 20% en poids, en particulier de 0,5 à 5% en poids, par rapport à la formulation de base.

**24.** Procédé selon la revendication 22 ou 23, dans lequel les substances d'augmentation de la viscosité comprennent des éthers et des esters de la cellulose, des dérivés polyvinyliques, l'alcool polyvinylique, des alginates, le xanthane, des pectines, des polyacrylates, des poloxamères et des poloxamines.

**25.** Procédé selon l'une quelconque des revendications 22 à 24, dans lequel l'excipient de médicament comprend en outre du sucre ou des alcools de sucre, en particulier le glucose, le mannose, le tréhalose, le mannitol et le sorbitol.

**26.** Procédé selon l'une quelconque des revendications 22 à 25, dans lequel l'excipient de médicament comprend en outre des porteurs de charges.

**27.** Procédé selon l'une quelconque des revendications 1 à 26, dans lequel les particules sont mises en dispersion dans de l'eau distillée, ou dans un milieu aqueux ou dans un milieu aqueux avec des adjuvants faits d'électrolytes, de monosaccharides et de disaccharides, de polyols ou de leurs mélanges.

**28.** Procédé selon la revendication 27, dans lequel les adjuvants comprennent le chlorure de sodium, le mannose, le glucose, le fructose, le xylose, le mannitol, le sorbitol, le xylitol et la glycérine.

**29.** Procédé selon l'une quelconque des revendications 1 à 28, dans lequel les particules sont soumises à une lyophilisation ou à un séchage par pulvérisation.

**30.** Procédé selon l'une quelconque des revendications 1 à 29, dans lequel l'excipient de médicament comprend en outre un ou plusieurs agents actifs.

**31.** Procédé selon la revendication 30, dans lequel, dans le cas de plusieurs agents actifs, un agent actif ou plusieurs agents actifs sont dissous l'un ou les uns dans les autres (sous la forme de ce que l'on appelle une solution solide) ou sont mis en dispersion (sous la forme de ce que l'on appelle une dispersion solide), sont absorbés à leur surface ou sont mis en dispersion dans la particule en tant que solution.

**32.** Procédé selon l'une quelconque des revendications 1 à 26, dans lequel les particules sont mises en dispersion dans un milieu non aqueux.

**33.** Procédé selon la revendication 32, dans lequel les particules sont mises en dispersion dans un milieu liquide, semi-solide ou solide.

**34.** Procédé selon la revendication 33, dans lequel les particules sont mises en dispersion dans un milieu huileux liquide comme l'huile de ricin, l'huile de noix, l'huile d'olive, l'huile neutre (produit Miglyol 812), l'huile de sésame, l'huile de maïs, l'huile de coton, l'huile d'amande, des triglycérides à chaîne moyenne ou d'autres huiles.

35. Procédé selon la revendication 33, dans lequel le milieu se compose de lipides ou de lipoïdes ou de leurs mélanges.

36. Procédé selon la revendication 35, dans lequel le milieu se compose de mono-, de di- ou de triglycérides (par exemple les produits Witepsole, Softisane), de cires, d'alcools gras ou d'esters d'alcools gras, de la cire d'abeille, de l'ester oléylique de l'acide oléique, du myristate d'isopropyle, de la cire de laine ou de leurs mélanges.

37. Procédé selon la revendication 33, dans lequel le milieu se compose de molécules organiques à longue chaîne ou de polymères, de polyéthylèneglycols liquides, semi-solides ou solides, de poloxamères, de poloxamines ou de leurs mélanges.

38. Procédé selon l'une quelconque des revendications 1 à 37, dans lequel le médicament ou le mélange de médicaments est soumis à une mouture afin de former une poudre, est mis en dispersion dans un milieu de dispersion, en particulier dans l'eau ou dans un milieu aqueux, et est alors soumis à une homogénéisation haute pression.

Figur 1

**Figur 2**

EP 0 790 821 B1

Figur 3

27

Figur 4

EP 0 790 821 B1

EP 0 790 821 B1

Figur 5

**Figur 6**

Figur 7

Figur 8

Figur 9

EP 0 790 821 B1

**Number**

Number

40*10³

35*10³

30*10³

25*10³

20*10³

15*10³

10*10³

5*10³

0

MESSUNG1
MESSUNG2
MESSUNG3

1

2

3

1   2   4   6   8   10   20   40

**Particle Diameter (um)**

**LC= 0.747 um   UC= 22.01 um (293.5*10³)**

Figur 10

Figur 11

EP 0 790 821 B1

Figur 12

Figur 13

Figur 14

Figur 15

EP 0 790 821 B1

Figur 16

EP 0 790 821 B1

Figur 17

41